# EUROPEAN PATENT APPLICATION

(11) **EP 4 799 643 A1**
(43) Date of publication of application: **02.09.2026**
(21) Application number: 24881654.8
(22) Date of filing: 23.10.2024
(51) Int. Cl.: A61K 45/06, A61P 1/16

(54) **PHARMACEUTICAL COMPOSITION AND USE THEREOF**

(30) Priority: 27.10.2023 CN 202311411787
(71) Applicant: Suzhou Ribo Life Science Co., Ltd., Suzhou, Jiangsu 215300 (CN)
(72) Inventor: GAO, Shan, Suzhou, Jiangsu 215300 (CN); GAN, Li Ming, Suzhou, Jiangsu 215300 (CN); LIANG, Zicai, Suzhou, Jiangsu 215300 (CN); ZHENG, Shuquan, Suzhou, Jiangsu 215300 (CN)
(74) Representative: SSM Sandmair
(86) International application number: PCT/CN2024/126793
(87) International publication number: WO 2025/087290

(57) **Abstract**

Provided is a pharmaceutical composition, comprising a pharmaceutical active component. The pharmaceutical active component consists of an RNAi agent and an immune response regulator. The RNAi agent and the immune response regulator exist independently; the RNAi agent refers to one or more of an siRNA composition, an siRNA conjugate, and a pharmaceutically acceptable salt thereof; the siRNA composition comprises an siRNA and a pharmaceutically acceptable carrier; the siRNA conjugate comprises an siRNA group and a conjugated group conjugatively linked to the siRNA group; the siRNA group refers to a group formed by removing one or more atoms or groups from the siRNA, and the siRNA is an siRNA capable of inhibiting HBV mRNA; based on siRNA, the weight ratio of the RNAi agent to the immune response regulator is (0.5-5000): 1. Further provided is a method for treating HBV-related diseases with the RNAi agent and the immune response regulator.

## Description

### TECHNICAL FIELD

The present disclosure relates to a pharmaceutical composition and use thereof, and in particular to a pharmaceutical composition for treating a disease associated with hepatitis B virus infection and use thereof.

### BACKGROUND

Viral hepatitis B (also known as hepatitis B) caused by infection with HBV virus is a type of infectious disease that poses a serious threat to the world, particularly China. Chronic HBV infection (CHB) can also lead to an increased probability of chronic hepatitis, cirrhosis, liver failure, and hepatocellular carcinoma (HCC).

At present, the two main drugs for treating hepatitis B are interferons and nucleoside analogs, but the two types of drugs have the problems of susceptibility to drug resistance after use, susceptibility to adverse reactions after use of interferons, susceptibility to drug resistance after use of nucleoside drugs, and relapse after discontinuation of medication.

In recent years, emerging small nucleic acid drugs have also shown high activity and persistence in inhibiting hepatitis B virus.

However, in the prior art, there are still no drugs and treatment methods for functionally curing hepatitis B disclosed.

### SUMMARY

The inventors of the present disclosure have found that a composition comprising an RNAi agent and an immune response regulator surprisingly shows superior therapeutic efficacy when used to clear HBsAg in a subject and/or to treat HBV infection. Therefore, the inventors made the following inventions.

In one aspect, the present disclosure provides a pharmaceutical composition comprising a pharmaceutically active component, wherein the pharmaceutically active component consists of an RNAi agent and an immune response regulator, and the RNAi agent and the immune response regulator exist independently; the RNAi agent refers to one or more of an siRNA composition, an siRNA conjugate, and a pharmaceutically acceptable salt thereof, the siRNA composition comprises an siRNA and a pharmaceutically acceptable carrier; the siRNA conjugate comprises an siRNA group and a conjugating group conjugatively linked to the siRNA group; the siRNA group refers to a group formed by removing one or more atoms or groups from the siRNA, and the siRNA is an siRNA capable of inhibiting HBV mRNA; based on siRNA, the weight ratio of the RNAi agent to the immune response regulator is (0.5-5000):1.

In another aspect, the present disclosure provides use of the pharmaceutical composition described herein in preparing a medicament for treating a disease associated with hepatitis B virus infection.

In yet another aspect, the present disclosure provides a method for treating a disease associated with hepatitis B virus infection, comprising administering to a subject an effective amount of the pharmaceutical composition described herein.

In yet another aspect, the present disclosure further provides a kit comprising the pharmaceutical composition described herein.

### Incorporation by Reference

All publications, patents, and patent applications mentioned in this specification are incorporated herein by reference to the same extent as if each individual publication, patent, or patent application was specifically and individually indicated to be incorporated herein by reference.

### Beneficial effects

Compared with administration of an RNAi agent or an immune response regulator alone, the pharmaceutical composition and the treatment method of the present disclosure exhibit an excellent anti-hepatitis B virus effect, have the potential to achieve a functional cure of hepatitis B virus, and exhibit significant synergistic utility and significantly better therapeutic effects. A higher reduction rate of HBsAg, HBeAg and HBV DNA content in serum can be achieved while administering a lower dose of drugs to a subject, and the production of anti-HBV antibodies in the subject can be promoted.

For example, compared to use of an siRNA conjugate or an immune response regulator alone, use of the pharmaceutical composition of the present disclosure was able to further reduce the HBV DNA level within the experimental period of up to 78 days, and the maximum HBV DNA inhibition rate reached 99.9992%. Moreover, the reduction in the HBV DNA was surprisingly greatly beyond the sum of the inhibition effects of an siRNA conjugate or an immune response regulator alone. Compared to use of the RNAi agent alone, the HBV DNA level could be further greatly reduced by 99.9% on the basis that the HBV DNA level had been greatly reduced in the RNAi agent alone group. Further results showed that in 6 experimental animals administered with the pharmaceutical composition of the present disclosure, the HBV DNA level of 1 experimental animal was reduced to the limit of detection (10^{3.18} IU/mL) or less on day 78. For another example, the present disclosure further verifies the inhibition effect on HBV DNA after administration of a lower dose of the pharmaceutical composition of the RNAi agent and the immune response regulator to mice. The results showed that HBV DNA was maintained at a relatively low level for up to 85 days after the first administration, with a maximum reduction of 4.48 log₁₀ IU/mL, i.e., the maximum HBV DNA inhibition rate reached 99.9967%. In addition, compared with the inhibition level when the RNAi agent was administered at a single dose of 9 mpk, the HBV DNA level in the mice administered with the pharmaceutical composition of the RNAi agent at a single dose of 3 mpk and the immune response regulator was further greatly reduced, with a maximum reduction of 2.76 log₁₀ IU/mL, i.e., the HBV DNA level was further reduced by 99.83%.

For another example, compared to use of an siRNA conjugate or an immune response regulator alone, use of the pharmaceutical composition of the present disclosure could further perform a reduction within the experimental period of up to 78 days, and the maximum HbsAg inhibition rate reached 99.9930%. Moreover, the reduction was surprisingly greatly beyond the sum of the inhibition effects of an siRNA conjugate or an immune response regulator alone, and could further reduce by 97.76% on the basis of the already greatly reduced HbsAg level in the conjugate group compared to use of the RNAi agent alone. Still further, on day 78, the HbsAg levels in some of the experimental animals in the test groups administered with the composition of the present disclosure were already below the limit of detection, indicating very excellent inhibition effects. For another example, the present disclosure further verifies the inhibition effect on HbsAg after administration of a lower dose of the pharmaceutical composition of the RNAi agent and the immune response regulator to mice. The results showed that HbsAg was maintained at a relatively low level for up to 85 days after the first administration, with a maximum reduction of 3.32 log₁₀ IU/mL, i.e., the maximum HbsAg inhibition rate reached 99.9521%. In addition, compared with the inhibition level when the RNAi agent was administered at a single dose of 9 mpk, the HbsAg level in the mice administered with the pharmaceutical composition of the RNAi agent at a single dose of 3 mpk and the immune response regulator was further greatly reduced, with a maximum reduction of 1.31 log₁₀ IU/mL, i.e., the HbsAg level was further reduced by 95.1022%.

For another example, compared to use of an siRNA conjugate or an immune response regulator alone, use of the pharmaceutical composition of the present disclosure could further reduce the HBeAg level within the experimental period of up to 78 days, and the reduction was beyond the inhibition effect of the siRNA conjugate or the immune response regulator used alone. The pharmaceutical composition of the present disclosure showed a further reduction of about 0.3 log₁₀ IU/mL compared with the RNAi agent used alone. For another example, the present disclosure further verifies that after a lower dose of the pharmaceutical composition of the RNAi agent and the immune response regulator was administered to mice, the HbeAg level in the mice also showed a further reduction of about 0.325 log₁₀ IU/mL than that of the RNAi agent drug administered alone at a relatively high dose.

For another example, the pharmaceutical composition of the present disclosure can induce the production of significant HBsAb in serum in mice, indicating that the pharmaceutical composition can not only effectively inhibit HBV antigens and DNA, but also stimulate the immune response in mice, showing excellent prospects for achieving a functional cure of hepatitis B.

### BRIEF DESCRIPTION OF THE DRAWINGS

FIG. 1 shows a line graph showing changes in the level of HBV DNA in the serum of HBV transgenic mice over time after in vivo administration of a control group or the pharmaceutical composition of the present disclosure.
FIG. 2 shows a line graph showing changes in the level of HbsAg in the serum of HBV transgenic mice over time after in vivo administration of a control group or the pharmaceutical composition of the present disclosure.
FIG. 3 shows a line graph showing changes in the level of HbeAg in the serum of HBV transgenic mice over time after in vivo administration of a control group or the pharmaceutical composition of the present disclosure.
FIG. 4 shows a line graph showing changes in the level of HBV DNA in the serum of HBV transgenic mice over time after in vivo administration of a control group or the pharmaceutical composition of the present disclosure.
FIG. 5 shows a line graph showing changes in the level of HbsAg in the serum of HBV transgenic mice over time after in vivo administration of a control group or the pharmaceutical composition of the present disclosure.
FIG. 6 shows a line graph showing changes in the level of HbeAg in the serum of HBV transgenic mice over time after in vivo administration of a control group or the pharmaceutical composition of the present disclosure.

### DETAILED DESCRIPTION

Specific embodiments of the present disclosure are described in detail below. It will be appreciated that the specific embodiments described herein are intended to illustrate and explain the present disclosure only rather than limit the present disclosure.

### Definitions

Unless otherwise specified, the nouns or terms used in the present disclosure have the meanings described below.

HBV DNA refers to a DNA sequence having a sequence shown in Genbank accession No. NC_003977.1. Further, unless otherwise specified, the "HBV mRNA" as used herein refers to the mRNA transcribed from the HBV DNA described above. The complete coding sequence of the reference sequence of the HBV genome can be found in, for example, GenBank Accession Nos. GI:21326584 and GI:3582357. It is well known to those skilled in the art that, based on the comparison of whole-gene nucleotide sequences, HBV can be divided into 9 subtypes: A, B, C, D, E, F, G, H and I. These 9 HBV subtypes are all within the scope of the HBV DNA described herein.

The uppercase letters C, G, U, and A represent the base composition of nucleotides; the lowercase letter m indicates that the nucleotide adjacent to the letter m on the left side is a methoxy modified nucleotide; the lowercase letter f indicates that the nucleotide adjacent to the letter f on the left side is a fluoro modified nucleotide; the lowercase letter s indicates that the two nucleotides adjacent to the letter s on the left and right sides are linked by a phosphorothioate group; P1 indicates that the nucleotide adjacent to the P1 on the right side is a 5'-phosphate nucleotide or 5'-phosphate analog modified nucleotide. In some embodiments, P1 is VP, Ps or P that indicates a specific modification, wherein the letter combination VP indicates that the nucleotide adjacent to the letter combination VP on the right side is a 5'-(E)-vinylphosphonate (E-VP) modified nucleotide, the letter combination Ps indicates that the nucleotide adjacent to the letter combination Ps on the right side is a phosphorothioate modified nucleotide, and the uppercase letter P indicates that the nucleotide adjacent to the letter P on the right side is a 5'-phosphate nucleotide.

"Fluoro modified nucleotide" refers to a nucleotide formed by substituting a 2'-hydroxy of a ribose group of the nucleotide with a fluoro, and "non-fluoro modified nucleotide" refers to a nucleotide formed by substituting the 2'-hydroxy of the ribose group of the nucleotide with a non-fluoro group, or a nucleotide analogue. "Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, e.g., an isonucleotide, a bridged nucleic acid (BNA for short) or an acyclic nucleotide. "Methoxy modified nucleotide" refers to a nucleotide formed by substituting the 2'-hydroxy of the ribose group with a methoxy group.

"Complementary" and "reversely complementary" are used interchangeably and have the meaning well known to those skilled in the art, that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (G) is always paired with the pyrimidine base cytosine (C). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mispairing" in the art means that in a double-stranded nucleic acid, the bases in the corresponding positions are not paired in a complementary manner.

"Substantially reversely complementary" means that there are no more than 3 base mispairings between two nucleotide sequences. "Substantially reverse complementary" means that there is no more than 1 base mispairing between two nucleotide sequences. "completely reverse complementary" means that there is no base mispairing between two nucleotide sequences.

"Nucleotide difference" between one nucleotide sequence and another nucleotide sequence means that the former has a change in the base type of the nucleotide at the same position as compared to the latter. For example, when one nucleotide base in the latter is A and the corresponding nucleotide base at the same position in the former is U, C, G, or T, it is considered that a nucleotide difference exists at that position between the two nucleotide sequences. In some embodiments, the replacement of a nucleotide at its original position with an abasic nucleotide or an equivalent thereof can also be considered that there is a nucleotide difference at that position.

In the context of the present disclosure, particularly when describing the preparation method for siRNA or siRNA conjugate in the pharmaceutical composition and/or RNAi agent of the present disclosure, unless otherwise specified, the nucleoside monomer refers to a modified or unmodified RNA/nucleoside phosphoramidites used in phosphoramidite solid-phase synthesis according to the type and sequence of nucleotides in the siRNA or siRNA conjugate to be prepared. The phosphoramidite solid phase synthesis is a method used in RNA synthesis well known to those skilled in the art. Unless otherwise specified, the nucleoside monomers used in the present disclosure are all commercially available or can be prepared by methods well known to those skilled in the art.

"Conjugation" refers to two or more chemical moieties each with specific function being linked to each other via a covalent linkage. Accordingly, a "conjugate" refers to a compound formed by covalent linkage of individual chemical moieties. Further, an "siRNA conjugate" refers to a compound formed by covalently linking one or more chemical moieties with specific functions to an siRNA. The siRNA conjugate should be understood as a general term of multiple siRNA conjugates or an siRNA conjugate represented by a certain chemical formula according to the context. In the context of the present disclosure, a "conjugating molecule" should be understood as a specific compound that may be conjugated to an siRNA through a reaction, ultimately forming a so-called siRNA conjugate. The "siRNA conjugate" as used in the present disclosure comprises an siRNA group and a conjugating group moiety, wherein the siRNA group refers to a chemical moiety formed from an siRNA molecule after one or more atoms are removed. It will be understood by those skilled in the art that the removal of the one or more atoms described above does not destroy the inhibition activity or stability of the siRNA against the target mRNA. For example, the siRNA group may be a chemical moiety formed in the siRNA after the hydrogen atom in the phosphoester bond is removed, or a chemical moiety formed in the siRNA after the hydrogen atom in the 5' hydroxy of the 5' terminal nucleotide of the sense strand or antisense strand is removed, or a chemical moiety formed in the siRNA after the hydrogen atom in the 3' hydroxy of the 3' terminal nucleotide of the sense strand or antisense strand is removed.

Those skilled in the art will appreciate that for any group containing one or more substituents, these groups are not intended to introduce any substitution group or substitution pattern that is sterically impractical, synthetically infeasible, and/or inherently unstable.

"Alkyl" refers to linear and branched chains having a specified number of carbon atoms, typically 1 to 20 carbon atoms, such as 1 to 10 carbon atoms, for example, 1 to 8 or 1 to 6 carbon atoms. For example, C₁-C₆ alkyl includes linear and branched chain alkyl groups of 1 to 6 carbon atoms. When reference is made to an alkyl residue having a specific number of carbons, all branched and linear forms having that number of carbons are intended to be encompassed. Therefore, for example, "butyl" is meant to include n-butyl, sec-butyl, isobutyl and t-butyl; and "propyl" includes n-propyl and isopropyl. Alkylene is a subset of alkyl and refers to residues which are identical to alkyl but have two attachment positions.

"Alkenyl" refers to an unsaturated branched or linear hydrocarbon group having at least one carbon-carbon double bond obtained by removing a molecule of hydrogen from adjacent carbon atoms of the parent alkyl group. The group may be in the cis or trans configuration of the double bond. Typical alkenyl groups include, but are not limited to: vinyl; propenyl, such as prop-1-en-1-yl, prop-1-en-2-yl, prop-2-en-1-yl (allyl), and prop-2-en-2-yl; and butenyl, such as but-1-en-1-yl, but-1-en-2-yl, 2-methylprop-1-en-1-yl, but-2-en-1-yl, but-2-en-2-yl, buta-1,3-dien-1-yl, buta-1,3-dien-2-yl, and the like. In certain embodiments, alkenyl groups have 2 to 20 carbon atoms, while in other embodiments, alkenyl groups have 2 to 10, 2 to 8, or 2 to 6 carbon atoms. Alkenylene is a subset of alkenyl and refers to residues which are identical to alkenyl but have two attachment positions.

"Alkynyl" refers to an unsaturated branched or linear hydrocarbon group having at least one carbon-carbon triple bond obtained by removing two molecules of hydrogen from adjacent carbon atoms of the parent alkyl group. Typical alkynyl groups include, but are not limited to: ethynyl; propynyl, such as prop-1-yn-1-yl and prop-2-yn-1-yl; and butynyl, such as but-1-yn-1-yl, but-1-yn-3-yl, but-3-yn-1-yl, and the like. In certain embodiments, alkynyl groups have 2 to 20 carbon atoms, while in other embodiments, alkynyl groups have 2 to 10, 2 to 8, or 2 to 6 carbon atoms. Alkynylene is a subset of alkynyl and refers to residues which are identical to alkynyl but have two attachment positions.

"Alkoxy" refers to an alkyl group of a specified number of carbon atoms linked by an oxygen bridge, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. An alkoxy group typically has 1 to 10, 1 to 8, 1 to 6, or 1 to 4 carbon atoms linked by an oxygen bridge.

"Aryl" refers to a group derived from an aromatic monocyclic or polycyclic hydrocarbon ring system by removing a hydrogen atom from a ring carbon atom. The aromatic monocyclic or polycyclic hydrocarbon ring system contains only hydrogen and carbons of 6 to 18 carbon atoms, wherein at least one ring in the ring system is fully unsaturated, i.e., a cyclic delocalized (4n+2)π-electron system according to the Hückel theory is included. Aryl groups include, but are not limited to, groups such as phenyl, fluorenyl, naphthyl, and the like. Arylene is a subset of aryl and refers to residues which are identical to aryl but have two attachment positions.

"Heteroaryl" refers to a group derived from a 3- to 18-membered aromatic ring and contains 2 to 17 carbon atoms and 1 to 6 heteroatoms selected from nitrogen, oxygen and sulfur. As used herein, a heteroaryl group may be a monocyclic, bicyclic, tricyclic or tetracyclic ring system, wherein at least one ring in the ring system is fully unsaturated, i.e., a cyclic delocalized (4n+2) π-electron system according to the Hückel theory is included. Heteroaryl groups include fused or bridged ring systems. In some embodiments, the heteroatoms in the heteroaryl group are oxidized heteroatoms. In some embodiments, one or more nitrogen atoms are included in the heteroaryl group. In some embodiments, one or more of the nitrogen atoms in the heteroaryl group are quaternized nitrogen atoms. A heteroaryl group is attached to the rest of the molecule via any ring atom. Examples of heteroaryl groups include, but are not limited to: azepinyl, acridinyl, benzimidazolyl, benzindolyl, 1,3-benzobisoxazolyl, benzofuranyl, benzooxazolyl, benzo[d]thiazolyl, benzothiadiazolyl, benzo[*b*][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[H]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pyridinyl and thiophenyl/thienyl.

A variety of hydroxy protecting groups may be used in the present disclosure. In general, protecting groups render a chemical functionality insensitive to specific reaction conditions and may be added and removed at that functionality in a molecule without substantially damaging the rest of the molecule. Representative hydroxy protecting groups are disclosed in Beaucage et al., Tetrahedron 1992, 48, 2223-2311, and Greene and Wuts, Protective Groups in Organic Synthesis, Chapter 2, 2nd ed, John Wiley & Sons, New York, 1991, each of which is hereby incorporated by reference in its entirety. In some embodiments, the protecting groups are stable under basic conditions and can be removed under acidic conditions. In some embodiments, non-exclusive examples of hydroxy protecting groups used herein include dimethoxytrityl (DMT), monomethoxytrityl, 9-phenyloxanthene-9-yl (Pixyl) and 9-(p-methoxyphenyl)xanth-9-yl (Mox). In some embodiments, non-exclusive examples of hydroxy protecting groups used herein include Tr (trityl), MMTr (4-methoxytrityl), DMTr (4,4'-dimethoxytrityl) and TMTr (4,4',4''-trimethoxytrityl).

The term "subject", as used herein, refers to any animal, such as a mammal or a marsupial. Subjects of the present disclosure include, but are not limited to, humans, non-human primates (e.g., rhesus or other types of macaques), mice, pigs, horses, donkeys, cows, rabbits, sheep, rats, and any species of poultry.

"Treatment" refers to means of obtaining a beneficial or desired result, including but not limited to therapeutic benefit. The term "therapeutic benefit" means eradicating or ameliorating the underlying disorder being treated. Furthermore, therapeutic benefit is obtained by eradicating or ameliorating one or more physiological symptoms associated with the underlying disorder, whereby improvement is observed in the subject, although the subject may still be afflicted by the underlying disorder.

"Hepatitis B surface antigen (HBsAg)" refers to a surface antigen main protein of hepatitis B virus (HBV), the meaning of which is well known to those skilled in the art, e.g., the protein with NCBI GENBANK data accession No. AAF24729.1. As used herein, "hepatitis B core antigen protein (HBcAg)" refers to the core antigen protein of HBV, the meaning of which is well known to those skilled in the art, e.g., the protein with NCBI GENBANK data accession No. AAO63517.1. As used herein. "Hepatitis B e antigen (HBeAg)" refers to the hepatitis B virus protein located between the envelope and the capsid of HBV, the meaning of which is well known to those skilled in the art, e.g., the protein with NCBI GENBANK data accession No. BAJ51621.1. It will be understood by those skilled in the art that in the amino acid sequences of the viral proteins described above, mutations or variations, including but not limited to, substitutions, deletions, and/or additions, such as HBsAg, HBcAg, and/or HBeAg of different genotypes or subtypes, may naturally occur or can be artificially introduced, and these mutations or variations do not affect biological functions thereof. All such natural or artificial variants are also included within the scope of the present disclosure.

### Pharmaceutical composition

In one aspect, the present disclosure provides a pharmaceutical composition comprising a pharmaceutically active component, wherein the pharmaceutically active component consists of an RNAi agent and an immune response regulator, and the RNAi agent and the immune response regulator exist independently; the RNAi agent refers to one or more of an siRNA composition, an siRNA conjugate, and a pharmaceutically acceptable salt thereof; the siRNA composition comprises an siRNA and a pharmaceutically acceptable carrier; the siRNA conjugate comprises an siRNA group and a conjugating group conjugatively linked to the siRNA group; the siRNA group refers to a group formed by removing one or more atoms or groups from the siRNA, and the siRNA is an siRNA capable of inhibiting HBV mRNA; based on siRNA, the weight ratio of the RNAi agent to the immune response regulator is (0.5-5000):1.

In the context of the present disclosure, the term "based on siRNA in the RNAi agent" in the siRNA composition refers to based on the siRNA contained in the composition. In the siRNA conjugate, based on the siRNA group, since the molecular weight of the siRNA group contained in the siRNA conjugate is substantially the same as that of the siRNA forming the siRNA group, the dose of the siRNA group in the siRNA conjugate is also described as "based on siRNA" for convenience.

In the pharmaceutical composition of the present disclosure, the pharmaceutically active component consists of the RNAi agent and the immune response regulator. The inventors of the present disclosure have surprisingly found that unlike the prior art, by containing an RNAi agent and an immune response regulator in a specific weight ratio, the pharmaceutical composition of the present disclosure can achieve an enhanced HBV inhibition effect without further use in combination with an HBV vaccine, and shows an excellent synergistic effect on HbsAg and HBV DNA inhibition. Thus, in some embodiments, in the pharmaceutical composition of the present disclosure, based on siRNA in the RNAi agent, the weight ratio of the RNAi agent to the immune response regulator is (0.5-2000):1 or (1-2000):1. In some embodiments, based on siRNA in the RNAi agent, the weight ratio of the RNAi agent to the immune response regulator is (1-400):1 or (2-400):1. In some embodiments, based on siRNA in the RNAi agent, the weight ratio of the RNAi agent to the immune response regulator is (2-150):1 or (3-150):1. In some embodiments, based on siRNA in the RNAi agent, the weight ratio of the RNAi agent to the immune response regulator is (2.4-50):1 or (4-50):1. The pharmaceutical composition of the present disclosure having the weight ratio described above is capable of better exerting the synergistic effect of the RNAi agent and the immune response regulator and producing a higher inhibition effect on HBV-related diseases or symptoms.

In some embodiments, the amount ratio of the RNAi agent to the immune response regulator in the pharmaceutical composition is calculated according to the dose. The amount of the RNAi agent in the pharmaceutical composition is calculated according to mg/kg body weight of the subject, and the amount of the immune response regulator is calculated according to the weight. In some embodiments, based on siRNA in the RNAi agent, the dose ratio of the RNAi agent to the immune response regulator is (0.02-540) mg/kg body weight of the subject:1 mg. In some embodiments, based on siRNA in the RNAi agent, the dose ratio of the RNAi agent to the immune response regulator is (0.03-400) mg/kg body weight of the subject:1 mg. In some embodiments, based on siRNA in the RNAi agent, the dose ratio of the RNAi agent to the immune response regulator is (0.06-380) mg/kg body weight of the subject:1 mg. In some embodiments, based on siRNA in the RNAi agent, the dose ratio of the RNAi agent to the immune response regulator is (0.03-180) mg/kg body weight of the subject:1 mg. In some embodiments, based on siRNA in the RNAi agent, the dose ratio of the RNAi agent to the immune response regulator is (0.06-180) mg/kg body weight of the subject:1 mg. In some embodiments, based on siRNA in the RNAi agent, the dose ratio of the RNAi agent to the immune response regulator is (0.1-80) mg/kg body weight of the subject:1 mg. In some embodiments, based on siRNA in the RNAi agent, the dose ratio of the RNAi agent to the immune response regulator is (0.2-30) mg/kg body weight of the subject:1 mg. Based on the doses of the RNAi agent and the immune response regulator for verifying the efficacy in animal models in the present disclosure, those skilled in the art can speculate about a dose range suitable for use in human subjects.

In some embodiments, the siRNA composition comprises an siRNA and a pharmaceutically acceptable carrier, wherein the siRNA is an siRNA in an siRNA conjugate.

In some embodiments, the siRNA conjugate in the pharmaceutical composition of the present disclosure comprises an siRNA group and a conjugating group conjugatively linked to the siRNA group.

In the pharmaceutical composition of the present disclosure, the "siRNA group" contained in the siRNA conjugate refers to a group formed after one or more atoms or groups are removed from the siRNA molecule to form a covalent linkage to the conjugating group. In some embodiments, the siRNA group is a group formed by removing 1 atom or 1 group from one or more nucleotide residues in the siRNA molecule. In some embodiments, the siRNA group is a group formed by removing 1 atom or 1 group from one or more nucleotide residues in the ribose ring, the base, or the phosphate group in the siRNA molecule. In some embodiments, the siRNA group is a group formed by removing 1 hydrogen atom or 1 hydroxy from the 3' and/or 5' terminal hydroxy in the sense strand or the antisense strand of the siRNA molecule. In some embodiments, the siRNA group is a group formed by removing 1 hydrogen atom from the 3' or 5' terminal hydroxy in the sense strand in the siRNA molecule. In some embodiments, the siRNA group is a group formed by removing 1 hydrogen atom in the 3' terminal hydroxy and/or the 5' terminal hydroxy in the sense strand in the siRNA molecule.

In some embodiments, the RNAi agent refers to an siRNA conjugate or a pharmaceutically acceptable salt thereof, wherein the conjugating group comprises a pharmaceutically acceptable targeting group and a linker; the siRNA group, the linker and the targeting group are linked sequentially, and each of the targeting groups is selected from ligands capable of binding to a hepatocyte surface receptor.

Generally, the conjugating group comprises at least one pharmaceutically acceptable targeting group and optionally a linker, and the siRNA group, the linker and the targeting group are linked sequentially. In some embodiments, the number of the targeting group is 1-6. In some embodiments, the number of the targeting group is 2-4. The siRNA group may be non-covalently or covalently conjugated to the conjugating group. For example, the same may be covalently conjugated to the conjugating group. The conjugating site between the siRNA group and the conjugating group may be at the 3' terminal or 5' terminal of the sense strand, or at the 5' terminal of the antisense strand in the siRNA group, or within the sequence of the siRNA group. In this context, "within the sequence" refers to nucleotides or internucleotide linkage groups other than the 3' and 5' terminal nucleotides of the sense and antisense strands of the siRNA. In some embodiments, the conjugating site between the siRNA group and the conjugating group is at the 3' terminal of the sense strand.

In some embodiments, the conjugating group may be linked to the phosphate group, the 2'-position hydroxy or the base of the siRNA group. In some embodiments, the conjugating group may be linked to the 3'-position hydroxy, and at this time, the nucleotides are linked by a 2'-5' phosphodiester bond. When the conjugating group is linked to the end of the siRNA group, the conjugating group is usually linked to the phosphate group or the 2'-position or 5'-position hydroxy of the nucleotide. When the conjugating group is linked to the internal sequence of the siRNA group, the conjugating group is usually linked to the ribose sugar ring or the base. For various linkage methods, please refer to Muthiah Manoharan et.al. siRNA conjugates carrying sequentially assembled trivalent N-acetylgalactosamine linked through nucleotides elicit robust gene silencing in vivo in hepatocytes. ACS Chemical biology, 2015, 10(5):1181-7.

In some embodiments, the siRNA group and the conjugating group may be linked by acid-labile or reducible chemical bonds. In the acidic environment of a cell endosome, these chemical bonds may degrade, thereby leaving the siRNA group in a free state. For example, the siRNA group may be reformed into the siRNA molecule to fully exert the RNA interference effect. For non-degradable conjugation methods, the conjugating group may be linked to the sense strand of the siRNA group to reduce the influence of conjugation on the inhibition activity of the siRNA group as much as possible.

The targeting group may be linked to the siRNA group through a suitable linker, and those skilled in the art may select a suitable linker according to the specific type of the targeting group. The types of these linkers, targeting groups and the linkage methods with the siRNA group, for example, may be found in the disclosure of WO2015006740A2, the entire contents of which are incorporated herein by reference.

In some embodiments, the targeting group may be a ligand conventionally used in the field of siRNA administration, e.g., various ligands described in WO2009082607A2, the entire disclosure of which is incorporated herein by reference.

In some embodiments, at least one or each of the targeting groups is selected from ligands capable of binding to receptors on the surfaces of cells expressing the target gene.

In some embodiments, at least one or each of the targeting groups is selected from ligands capable of binding to receptors on the surfaces of mammalian hepatocytes. In some embodiments, each of the targeting groups is independently a ligand that has an affinity for an asialoglycoprotein receptor (ASGPR) on the surface of mammalian hepatocytes. In some embodiments, each of the targeting groups is independently an asialoglycoprotein or a sugar. In some embodiments, each of the targeting groups is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, 2,3,4-tri-O-acetyl-1-thio-6-O-trityl-α-D-glucopyranoside methyl ester, 4-thio-β-D-galactopyranose, 3,4,6,7-tetra-O-acetyl-2-deoxy-1,5-dithio-α-D-glucopyranoside ethyl ester, 2,5-anhydro-D-allose nitrile, ribose, D-ribose, D-4-thioribose, L-ribose, and L-4-thioribose. In some embodiments, at least one or each of the targeting groups is galactose or N-acetylgalactosamine.

In some embodiments, the linker in the siRNA conjugate has a structure as represented by formula (301): 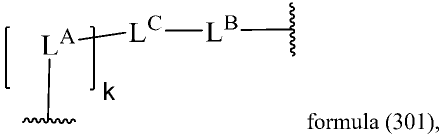
wherein k is an integer of 1-3;
L^{A} has a structure containing an amide bond represented by formula (302), L^{B} has a structure containing N-acylpyrrolidine represented by formula (303), containing carbonyl and oxygen atoms, and L^{C} is a linking group based on hydroxymethylaminomethane, dimethylolaminomethane or trishydroxymethylaminomethane; 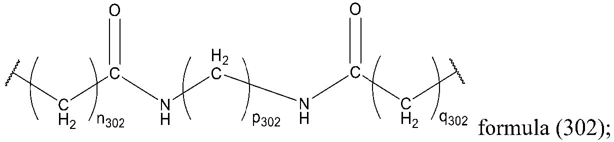 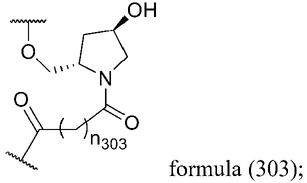
wherein n₃₀₂, q₃₀₂ and p₃₀₂ are each independently an integer of 2-6, and optionally, n₃₀₂, q₃₀₂ and p₃₀₂ are each independently 2 or 3; and n₃₀₃ is an integer of 4-16, and optionally, n₃₀₃ is an integer of 8-12. 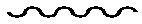 indicates the point at which the group is covalently linked.

In the linker, each L^{A} is separately linked to one of the targeting groups via an ether bond, and is linked to the L^{C} moiety by forming an ether bond via the oxygen atom of the hydroxy in the L^{C} moiety; L^{B} is linked by an amide bond formed by the carbonyl in formula (303) and the nitrogen atom of the amino in the L^{C} moiety, and linked via a phosphate bond or a phosphorothioate bond formed by the oxygen atom in formula (303) and the oxygen atom of the siRNA group.

In some embodiments, the RNAi agent refers to a conjugate having a structure represented by formula (305) or a pharmaceutically acceptable salt thereof: 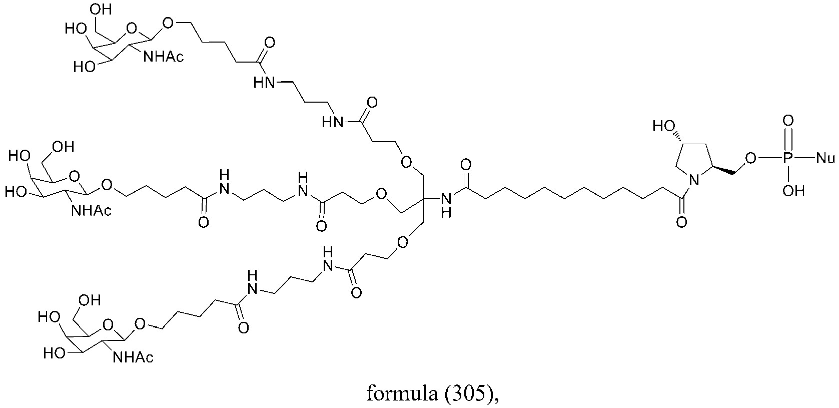 wherein Nu represents the siRNA group.

In some embodiments, the linker in the siRNA conjugate has a structure represented by formula (306): 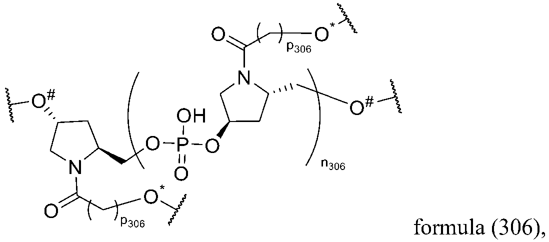 wherein n₃₀₆ is an integer of 0-3, each p₃₀₆ is independently an integer of 1-6, and 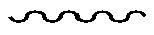 represents the site where the group is covalently linked; the linking group forms an ether bond linkage to the targeting group through the oxygen atom marked by *. The linking group forms a phosphoester bond or a phosphorothioate ester bond linkage through at least one of the oxygen atoms marked by # with the siRNA group, and the rest are linked to a hydrogen atom through the oxygen atom marked by # to form a hydroxy, or linked to a C₁-C₃ alkyl group to form a C₁-C₃ alkoxy group.

In some embodiments, the siRNA conjugate has a structure represented by formula (307): 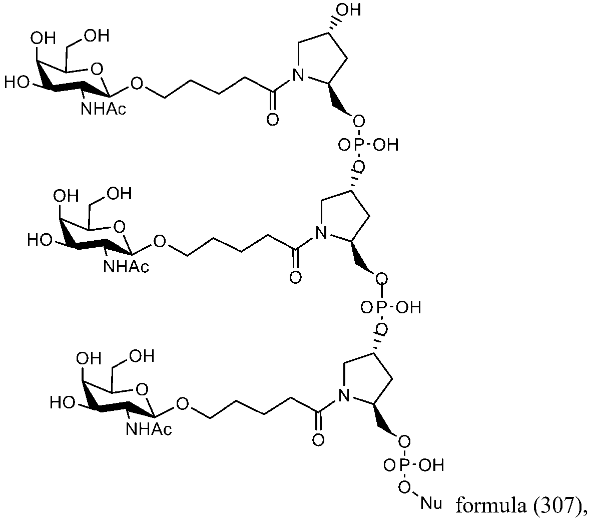 wherein Nu represents the siRNA group.

In some embodiments, the RNAi agent refers to a conjugate having a structure represented by formula (308) or a pharmaceutically acceptable salt thereof: 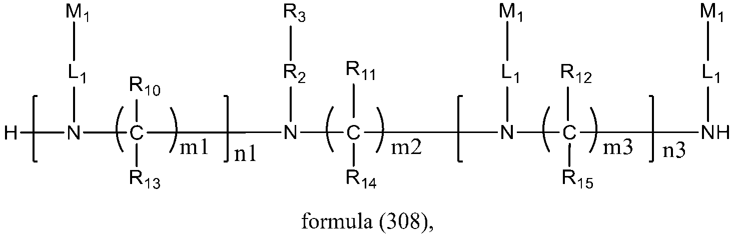 wherein,
n1 is an integer selected from 1-3, and n3 is an integer selected from 0-4;
each m1, m2, or m3 is independently an integer selected from 2-10;
R₁₀, R₁₁, R₁₂, R₁₃, R₁₄ or R₁₅ are each independently H, or are selected from the group consisting of the following groups: C₁-C₁₀ alkyl, C₁-C₁₀ haloalkyl and C₁-C₁₀ alkoxy;
R₃ has a structure represented by formula (A59): 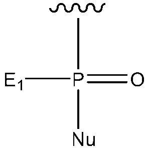 formula (A59),
wherein E₁ is OH, SH or BH₂, and Nu represents the siRNA group;
R₂ is a linear alkylene group with a length of 1-20 carbon atoms, wherein one or more carbon atoms are optionally replaced by any one or more selected from the group consisting of the following groups: C(O), NH, O, S, CH=N, S(O)₂, C₂-C₁₀ alkenylene, C₂-C₁₀ alkynylene, C₅-C₁₀ cyclohydrocabylene, C₆-C₁₀ arylene, C₃-C₁₈ heterocyclylene and C₅-C₁₀ heteroarylene; and wherein R₂ may optionally have any one or more substituents from the group consisting of the following groups: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halo substituents, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), - CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
each L₁ is independently a linear alkylene group with a length of 1-70 carbon atoms, wherein one or more carbon atoms are optionally replaced by any one or more selected from the group consisting of the following groups: C(O), NH, O, S, CH=N, S(O)₂, C₂-C₁₀ alkenylene, C₂-C₁₀ alkynylene, C₆-C₁₀ arylene, C₃-C₁₈ heterocyclylene and C₅-C₁₀ heteroarylene; and wherein L₁ may optionally have any one or more substituents from the group consisting of the following groups: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halo substituents, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), - CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
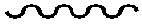 indicates the site where the group is covalently linked;
M₁ represents a targeting group, and its definition and optional range are the same as above. In some embodiments, each M₁ is independently selected from one of the ligands that have an affinity for the asialoglycoprotein receptor on the surface of mammalian hepatocytes.

Those skilled in the art will appreciate that although L₁ is defined as a linear alkyl group for convenience, it may not be a linear group or have a different name, such as amine or alkenyl as a result of the substitutions and/or replacements described above. For the purposes of the present disclosure, the length of L₁ is the number of atoms in the chain connecting the two points of attachment. For such purpose, a ring obtained by substituting a carbon atom of the linear alkylene group, such as a heterocyclylene or heteroarylene, is counted as one atom.

When M₁ is a ligand having affinity for asialoglycoprotein receptors on the surface of mammalian hepatocytes, in some embodiments, n1 may be an integer from 1-3, and n3 may be an integer from 0-4, such that the number of M₁ ligands in the conjugate is at least 2. In some embodiments, n1+ n3 ≥ 2, so that the number of M₁ ligands is at least 3, and the M₁ ligands more readily bind to the liver surface asialoglycoprotein receptor, which in turn facilitates the entry of the conjugate into the cell by endocytosis. Experiments have shown that when the number of M₁ ligands is greater than 3, the ease of binding of M₁ ligands to asialoglycoprotein receptors on the liver surface does not increase significantly. Therefore, from the perspective of ease of synthesis, structure/process cost and delivery efficiency and other aspects into consideration, in some embodiments, n1 is an integer of 1-2, n3 is an integer of 0-1, and n1 + n3 = 2-3.

In some embodiments, when m1, m2, and m3 are independently selected from an integer of 2-10, the spatial positions between multiple M₁ ligands may be suitable for the binding of M₁ ligands to asialoglycoprotein receptors on the liver surface. In order to make the conjugates provided by the present disclosure simpler, easier to synthesize and/or reduce costs, in some embodiments, m1, m2 and m3 are each independently an integer of 2-5, and in some embodiments, m1 = m2 = m3.

Those skilled in the art will understand that when R₁₀, R₁₁, R₁₂, R₁₃, R₁₄ and R₁₅ are each independently selected from one of H, C₁-C₁₀ alkyl, C₁-C₁₀ haloalkyl, and C₁-C₁₀ alkoxy, the purpose of the present disclosure may be achieved without changing the properties of the conjugates disclosed herein. In some embodiments, R₁₀, R₁₁, R₁₂, R₁₃, R₁₄ and R₁₅ are each independently selected from H, methyl, and ethyl. In some embodiments, R₁₀, R₁₁, R₁₂, R₁₃, R₁₄ and R₁₅ are all H.

In some embodiments, R₃ is a group with the structure represented by formula A59, wherein E₁ is OH, SH or BH₂. On the basis of the consideration of the availability of raw materials for preparation, in some embodiments, E₁ is OH or SH.

In some embodiments, R₂ is selected to achieve linkage to N and A59 on the nitrogenous backbone. In the context of the present disclosure, "nitrogenous backbone" refers to a chain structure in which carbon atoms linked to R₁₀, R₁₁, R₁₂, R₁₃, R₁₄ and R₁₅ are linked to N. Thus, R₂ may be any linking group capable of linking the A59 group to the N of the nitrogenous backbone in an appropriate manner. In some embodiments, in the case of preparing the siRNA conjugate by a solid phase synthesis process, the R₂ group needs to contain both the linking site linked to the N on the nitrogenous backbone and the linking site linked to the P in the R₃. In some embodiments, the site linked to N on the nitrogenous backbone in the R₂ group forms an amide bond with N, and the site linked to P on the R₃ group forms a phosphate bond with P. In some embodiments, the length of R₂ is 3-25 atoms, 3-20 atoms, 4-15 atoms, or 5-12 atoms. In some embodiments, R₂ comprises a first attachment position and an optional second functional group, wherein the first attachment position is an attachment position for forming a phosphoester bond or a phosphorothioate ester bond with an oligonucleotide or nucleotide, and the second functional group is a functional group formed after cleavage of the covalent linkage with a solid phase support. In some embodiments, R₂ is B5, B6, B5' or B6': 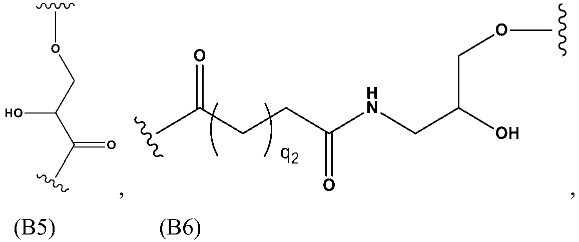 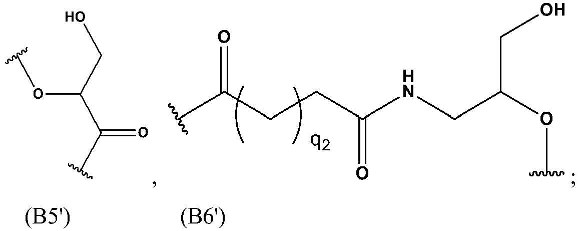 wherein 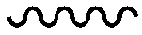 represents the site where the group is covalently linked.

The value range of q₂ may be an integer of 1-10, and in some embodiments, q₂ is an integer of 1-5.

The function of L₁ is to link the M₁ ligand to N on the nitrogenous backbone, providing a targeting function for the siRNA conjugate. In some embodiments, L₁ is selected from a combination of one or more of the groups in formulas A1-A26. In some embodiments, L₁ is selected from one of or a combination of two or more of the linkages A1, A4, A5, A6, A8, A10, A11 and A13. In some embodiments, L₁ is selected from a combination of at least 2 of the linkages A1, A4, A8, A10 and A11. In some embodiments, L₁ is selected from a combination of at least 2 of the linkages A1, A8, and A10.

In some embodiments, the length of L₁ may be 3-25 atoms, 3-20 atoms, 4-15 atoms, or 5-12 atoms. In some embodiments, the length of L₁ is 3, 4, 5, 6, 7, 8, 9, 10, 11, 12, 13, 14, 15, 16, 17, 18, 19, 20, 21, 22, 23, 24, 25, 30, 35, 40, 45, 50, 55 or 60 atoms.

In some embodiments, j1 is an integer of 2-10, and in some embodiments, j1 is an integer of 3-5. In some embodiments, j2 is an integer of 2-10, and in some embodiments, j2 is an integer of 3-5. R' is C1-C4 alkyl, and in some embodiments, R' is one of methyl, ethyl and isopropyl. Ra is one of A27, A28, A29, A30 and A31, and in some embodiments, Ra is A27 or A28. Rb is C1-C5 alkyl, and in some embodiments, Rb is one of methyl, ethyl, isopropyl and butyl. In some embodiments, each of j1, j2, R', Ra, and Rb in formulas A1-A26 is selected to achieve the linkage of the M₁ ligand to the N on the nitrogenous backbone, and to make the spatial position between the M₁ ligands more suitable for the M₁ ligand to bind to the liver surface asialoglycoprotein receptor.

In some embodiments, the siRNA conjugate has the structure represented by formula (403), (404), (405), (406), (407), (408), (409), (410), (411), (412), (413), (414), (415), (416), (417), (418), (419), (420), (421) or (422):

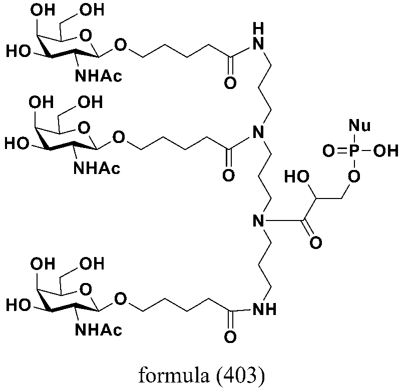

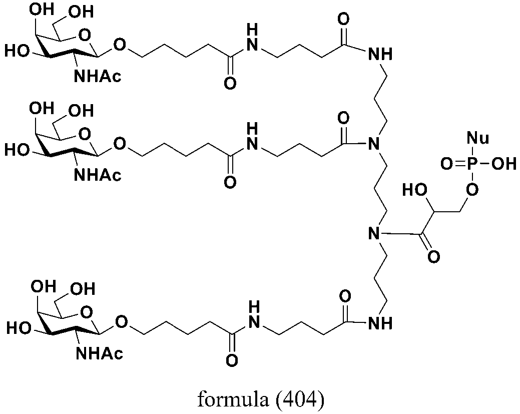

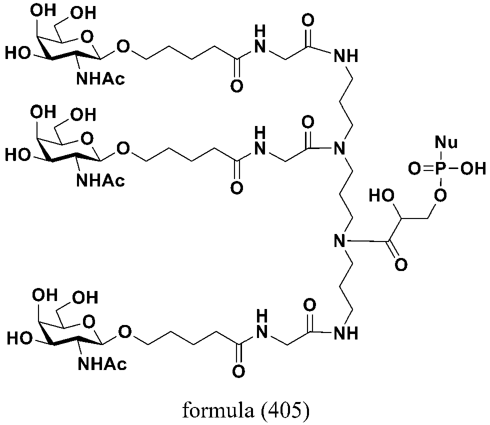

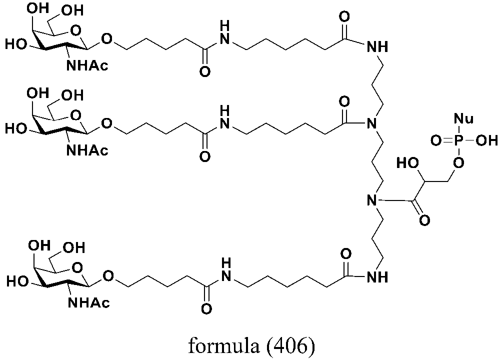

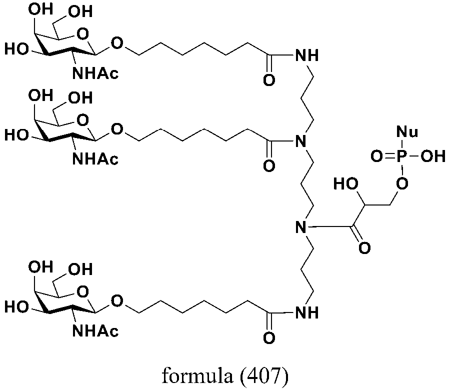

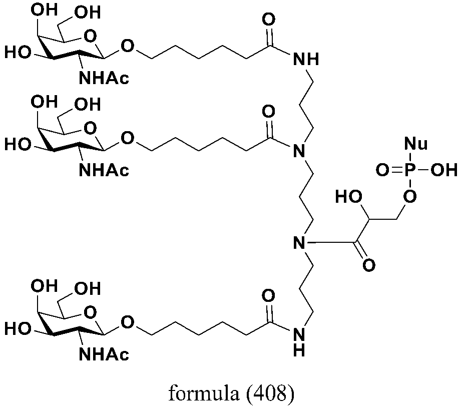

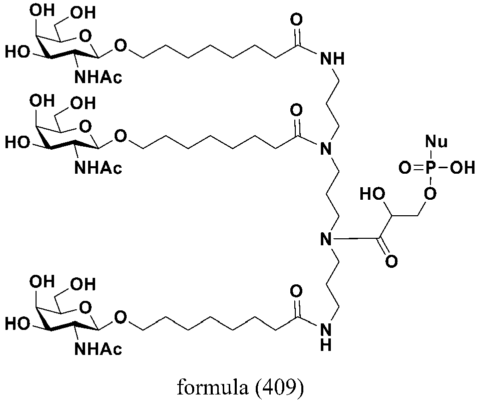

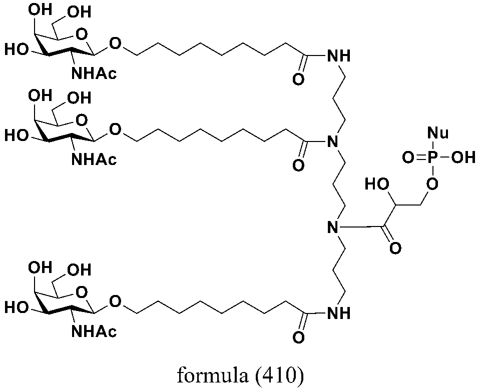

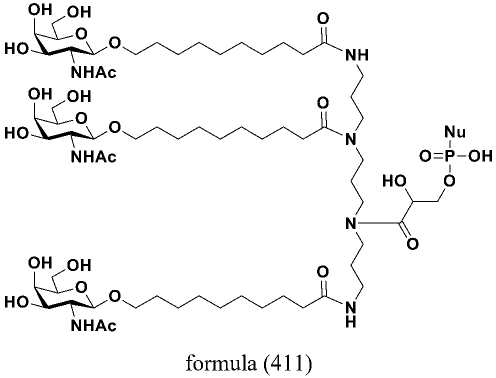

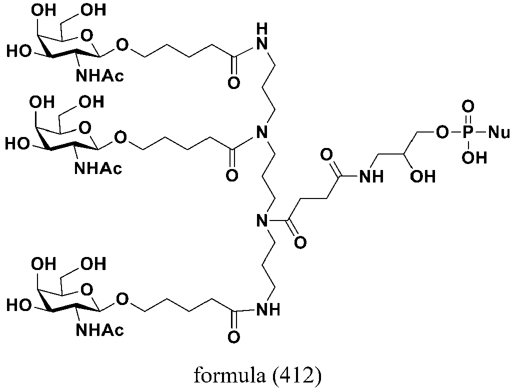

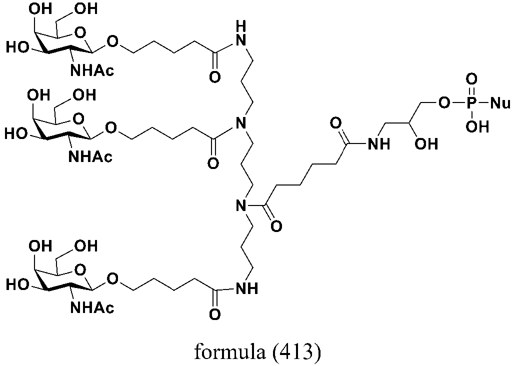

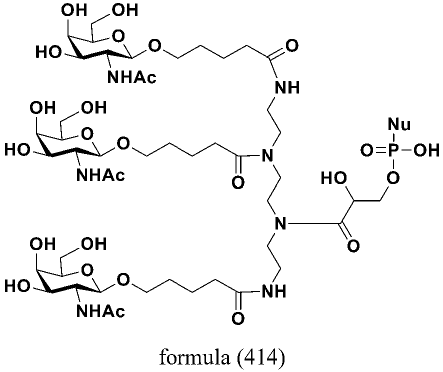

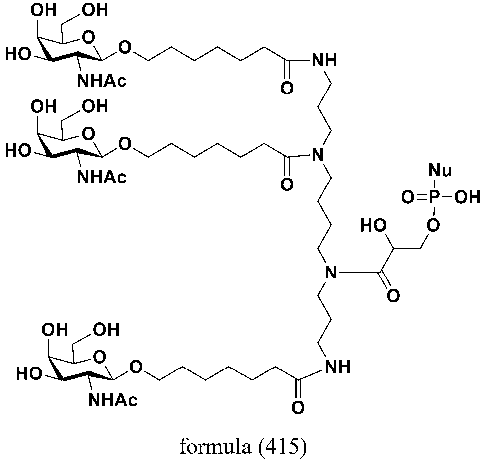

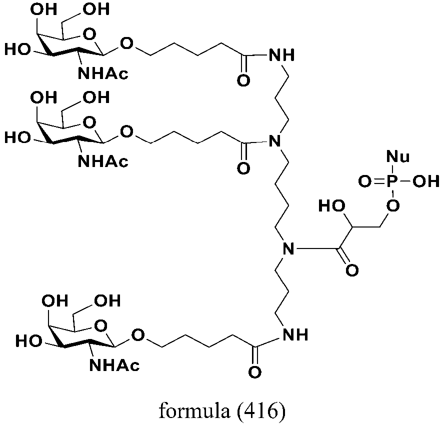

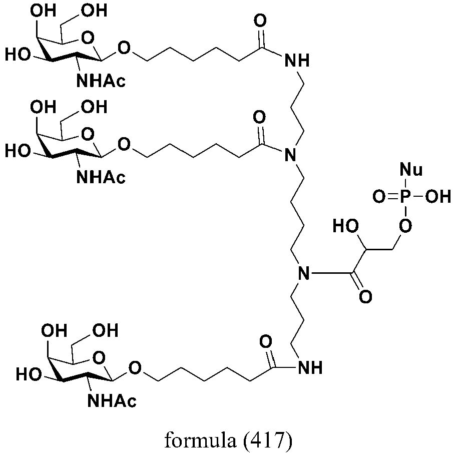

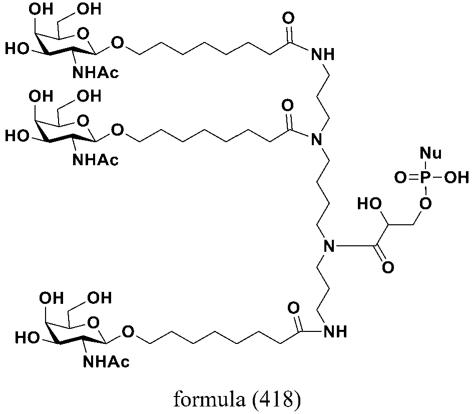

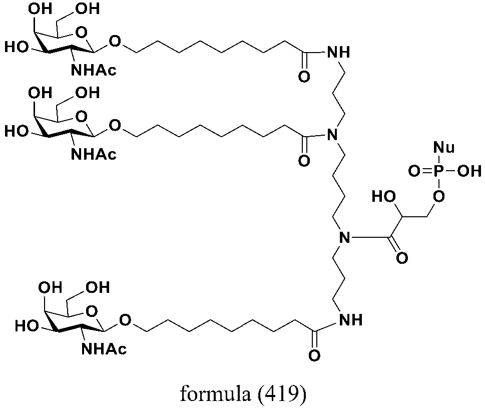

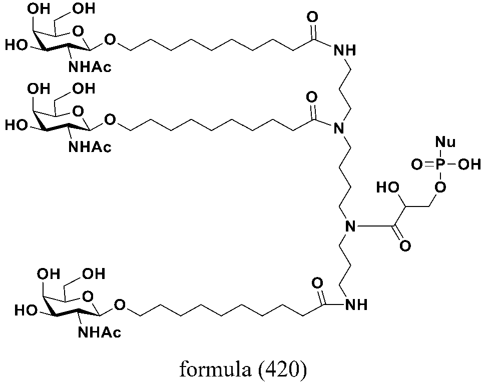

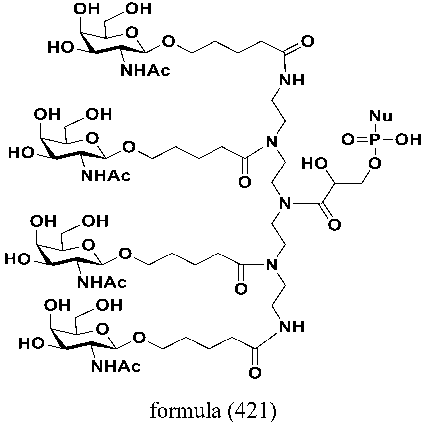

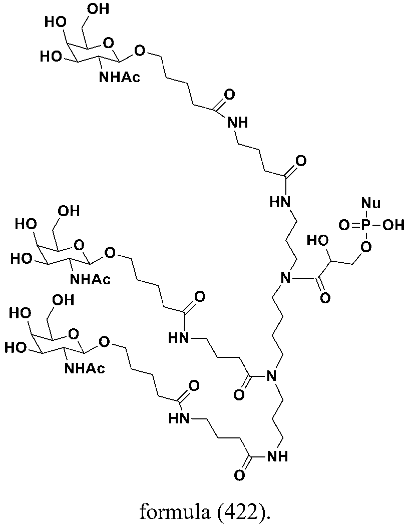

In some embodiments, P in formula (A59) may be linked to any possible position in the siRNA group. For example, P in formula (A59) may be linked to any nucleotide in the sense strand or the antisense strand in the siRNA group. In some embodiments, P in formula (A59) is linked to any nucleotide of the sense strand. In some embodiments, P in formula (A59) is linked to the end of the sense or antisense strand. In some embodiments, P in formula (A59) is linked to the end of the sense strand. The end refers to the first 4 nucleotides in the sense strand or the antisense strand from one end thereof. In some embodiments, P in formula (A59) is linked to the end of the sense or antisense strand in the siRNA group. In some embodiments, P in formula (A59) is linked to the 3' terminal of the sense strand. In the case of being linked to the above positions of the sense strand, after the siRNA conjugate enters the cell, upon unwinding, a separate antisense strand of the siRNA may be released to regulate target gene expression.

P in formula (A59) may be linked to any possible position on the nucleotide in the siRNA group, for example, the 5' position of the nucleotide, the 2' position of the nucleotide, the 3' position of the nucleotide or the base of the nucleotide. In some embodiments, P in formula (A59) may be linked to the 2' position, the 3' position or the 5' position of the nucleotides in the siRNA group by forming a phosphodiester bond. In some embodiments, P in formula (A59) is linked to the oxygen atom formed after removal of the hydrogen atom from the 3' hydroxy of the 3' terminal nucleotide of the sense strand, or P in formula (A59) is linked to the nucleotide by substituting the hydrogen in 2'-hydroxy of one nucleotide in the sense strand, or P in formula (A59) is linked to the nucleotide by substituting the hydrogen in 5'-hydroxy of the nucleotide at 5' terminal of the sense strand.

In some embodiments, the siRNA group contained in the siRNA conjugate may be formed by removing one or more atoms from the siRNA molecule described above. The siRNA and/or siRNA conjugate containing the sequences shown in SEQ ID NOs: 11-24 exhibit low off-target effects and high HBV mRNA inhibition activity. The siRNA conjugate also shows relatively high efficiency in entering cells. In some embodiments, the siRNA conjugate has a structure represented by formula (403) and the siRNA sequences shown in SEQ ID NOs: 11 and 12.

### siRNA composition capable of inhibiting HBV mRNA

The RNAi agent of the present disclosure may also be an siRNA composition capable of inhibiting HBV mRNA, the siRNA composition comprises the siRNA described above and a pharmaceutically acceptable carrier.

In some embodiments, the siRNA composition may be in the form of a liposomal formulation. In some embodiments, the pharmaceutically acceptable carrier used in the liposomal formulation comprises an amine-containing transfection compound (hereinafter also referred to as a critical lipid), a helper lipid and/or a pegylated lipid. The critical lipid, helper lipid and pegylated lipid may be selected from one or more of the amine-containing transfection compounds described in Chinese patent application CN103380113A (which is incorporated herein by reference in its entirety) or pharmaceutically acceptable salts or derivatives, helper lipids and pegylated lipids thereof.

In some embodiments, the critical lipid may be a compound represented by formula (201) as described in Chinese patent application CN103380113A or a pharmaceutically acceptable salt thereof: 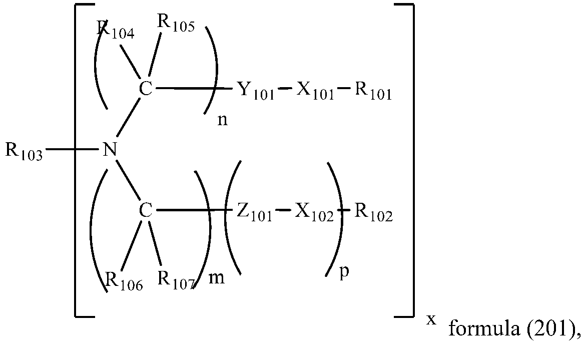 wherein:
X₁₀₁ and X₁₀₂ are each independently O, S, N-A or C-A, wherein A is hydrogen or a C₁-C₂₀ hydrocarbon chain;
Y₁₀₁ and Z₁₀₁ are each independently C=O, C=S, S=O, CH-OH or SO₂;
R₁₀₁, R₁₀₂, R₁₀₃, R₁₀₄, R₁₀₅, R₁₀₆ and R₁₀₇ are each independently hydrogen; a cyclic or acyclic, substituted or unsubstituted, branched or linear aliphatic group; a cyclic or acyclic, substituted or unsubstituted, branched or linear heteroaliphatic group; a substituted or unsubstituted, branched or linear acyl; a substituted or unsubstituted, branched or linear aryl; or a substituted or unsubstituted, branched or linear heteroaryl;
x is an integer of 1-10;
n is an integer of 1-3, m is an integer of 0-20, p is 0 or 1; wherein if m = p = 0, R₁₀₂ is hydrogen; and
if at least one of n or m is 2, R₁₀₃ and nitrogen in formula (201) form a structure represented by formula (202) or formula (203): 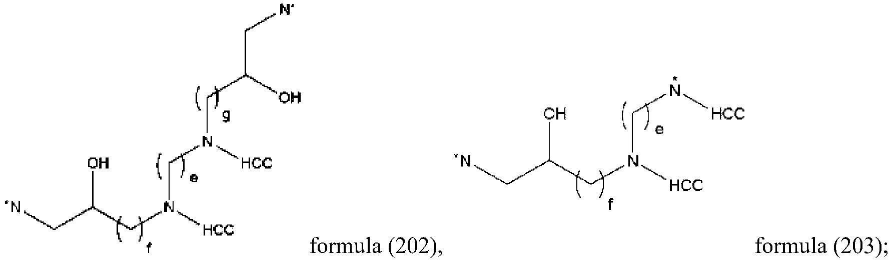
wherein g, e, and f are each independently an integer of 1-6, "HCC" represents a hydrocarbon chain, and each *N represents a nitrogen atom in formula (201).

In some embodiments, R₁₀₃ is a polyamine. In other embodiments, R₁₀₃ is a ketal. In some embodiments, each of R₁₀₁ and R₁₀₂ in formula (201) is independently any substituted or unsubstituted, branched or linear alkyl or alkenyl, the alkyl or alkenyl has 3 to about 20 carbon atoms, for example, 8 to about 18 carbon atoms, and 0 to 4 double bonds, for example, 0 to 2 double bonds.

In some embodiments, if each of n and m independently has a value of 1 or 3, R₁₀₃ may be any of formulas (204)-(213): 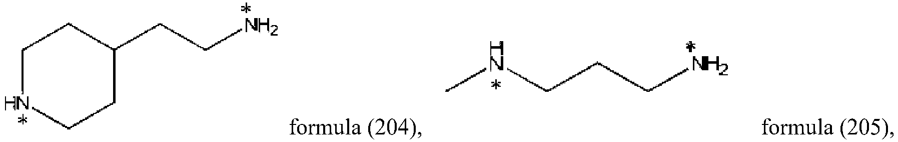 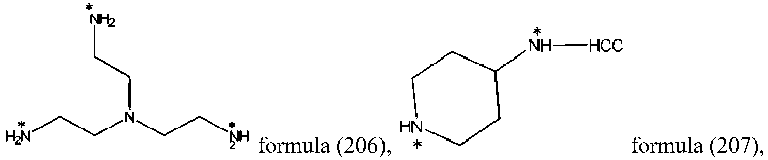 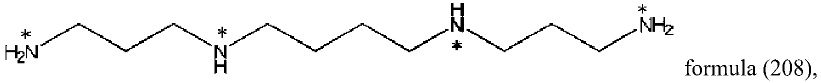 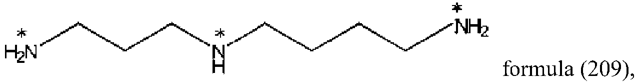 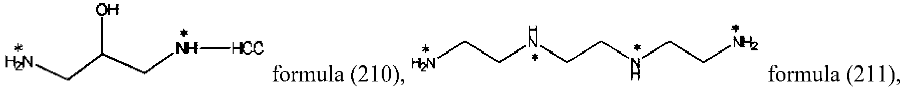 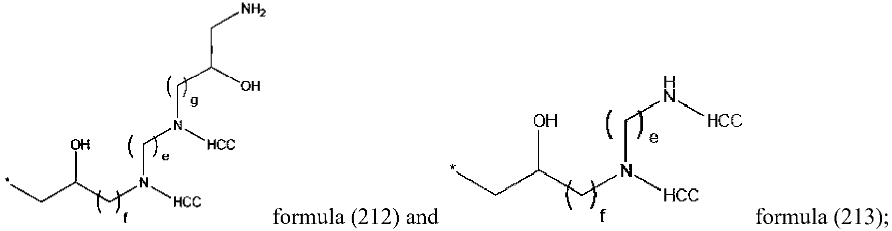 wherein in formula (204)-formula (213), g, e and f are each independently an integer of 1-6, each "HCC" represents a hydrocarbon chain, and each * shows possible attachment positions of R₁₀₃ to the nitrogen atom in formula (201), wherein each H at any * position may be substituted to achieve linkage to the nitrogen atom in formula (201).

The compound represented by formula (201) can be obtained by any reasonable method by those skilled in the art. In some embodiments, the compound represented by formula (201) may be prepared according to the description in Chinese patent application CN103380113A.

In some embodiments, the critical lipid is a critical lipid represented by formula (214) and/or a critical lipid represented by formula (215): 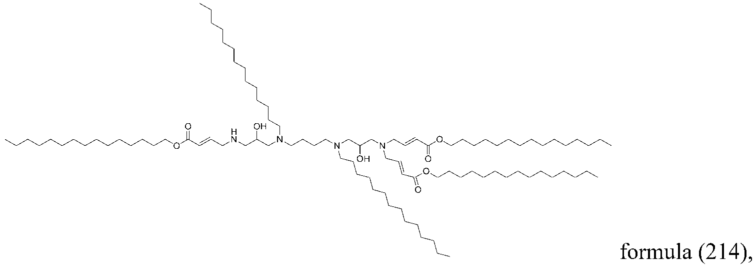 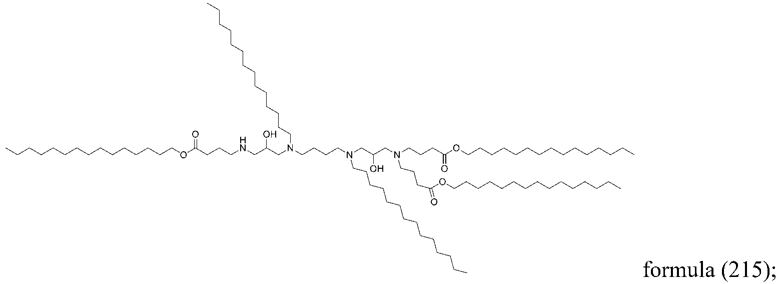
the helper lipid is a cholesterol, a cholesterol analog and/or a cholesterol derivative;
the pegylated lipid is 1,2-dipalmitoyl-sn-glycero-3-phosphatidylethanolamine-N-[methoxy(polyethylene glycol)]-2000.

In some embodiments, in the siRNA composition, the molar ratio among the critical lipid, the helper lipid and the pegylated lipid is (19.7-80) : (19.7-80) : (0.3-50), such as (50-70) : (20-40) : (3-20).

In some embodiments, the particles of the siRNA composition formed from the conjugate provided by the present disclosure and the amine-containing transfection reagent described above have an average diameter of about 30 nm to about 200 nm, typically about 40 nm to about 135 nm; more typically, the average diameter of the liposome particles is about 50 nm to about 120 nm, about 50 nm to about 100 nm, about 60 nm to about 90 nm or about 70 nm to about 90 nm. For example, the average diameter of the liposome particles is about 30, 40, 50, 60, 70, 75, 80, 85, 90, 100, 110, 120, 130, 140, 150 or 160 nm.

In some embodiments, in the siRNA composition formed from the conjugate provided by the present disclosure and the amine-containing transfection reagent described above, the weight ratio (weight/weight ratio) of the conjugate to all lipids (such as the critical lipids, helper lipids and/or pegylated lipids) is within the range of from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12 or from about 1:6 to about 1:10. For example, the weight ratio of the conjugate provided by the present disclosure to all lipids is about 1:5, 1:6, 1:7, 1:8, 1:9, 1:10, 1:11, 1:12, 1:13, 1:14, 1:15, 1:16, 1:17 or 1:18.

In some embodiments, each component of the siRNA composition may exist independently when sold, and may exist in the form of a liquid agent when used. In some embodiments, the siRNA composition of the present disclosure may be prepared according to various known methods, and may be prepared by substituting the existing nucleic acid component with the siRNA described above. In some embodiments, the composition may be prepared as follows:
the critical lipid, helper lipid and pegylated lipid are suspended in the alcohol according to the molar ratio as described above and mixed to obtain a lipid solution, wherein the amount of alcohol is such that the total mass concentration of the obtained lipid solution is 2-25 mg/mL; for example, it may be 8-18 mg/mL. The alcohol is selected from pharmaceutically acceptable alcohols, such as alcohols that are liquid at about room temperature, for example, one or more of ethanol, propylene glycol, benzyl alcohol, glycerin, polyethylene glycol 200, polyethylene glycol 300, polyethylene glycol 400, for example, it may be ethanol.

The siRNA is dissolved in a buffered salt solution to obtain an siRNA aqueous solution. The concentration of the buffered salt solution is 0.05-0.5 M, for example, 0.1-0.2 M; the pH of the buffered salt solution is adjusted to 4.0-5.5, for example, 5.0-5.2; the amount of the buffered salt solution makes the concentration of the siRNA do not exceed 0.6 mg/mL, for example, 0.2-0.4 mg/mL. The buffered salt is selected from one or more of soluble acetate and soluble citrate, for example, sodium acetate and/or potassium acetate.

The lipid solution and the siRNA aqueous solution are mixed, and the mixed product is incubated at 40-60 °C for at least 2 minutes, for example, 5-30 minutes, to obtain an incubated liposomal formulation. The volume ratio of the lipid solution to the conjugate aqueous solution is 1:(2-5), for example, 1:4.

The incubated liposomal formulation is concentrated or diluted, impurities are removed, and the agent is sterilized to obtain the siRNA composition provided by the present disclosure, whose physical and chemical parameters are pH of 6.5-8, encapsulation efficiency of not less than 80%, particle size of 40-200 nm, polydispersity index of not higher than 0.30, and osmotic pressure of 250-400 mOsm/kg. For example, the physical and chemical parameters may be pH of 7.2-7.6, encapsulation efficiency of not less than 90%, particle size of 60-100 nm, polydispersity index of not higher than 0.20, and osmotic pressure of 300-400 mOsm/kg.

The concentration or dilution may be performed before, after or simultaneously with the removal of impurities. Various existing methods may be used to remove impurities, for example, a tangential flow system and a hollow fiber column may be used for ultrafiltration at 100 KDa, wherein the ultrafiltration exchange solution is phosphate buffered saline (PBS) with pH of 7.4. Various existing methods may be used for the sterilization, for example, filtration sterilization on a 0.22 µm filter may be used.

It will be understood by those skilled in the art that in the RNAi agent, the siRNA and the siRNA conjugate in any suitable ratio can achieve the purpose of the present disclosure. For example, in some embodiments, the molar ratio of the siRNA to the siRNA conjugate may be 0:1000-1000:0. In some embodiments, in the RNAi agent, the molar ratio of the siRNA to the siRNA conjugate is 1:100-100:1, 1:50-50:1, or 1:10-10:1. In some embodiments, in the RNAi agent, the molar ratio of the siRNA to the siRNA conjugate is 1:5-5:1. In some embodiments, the RNAi agent is an siRNA conjugate.

### siRNA

In the pharmaceutical composition described in the present disclosure, the RNAi agent comprises an siRNA composition or an siRNA conjugate. In some embodiments, the siRNA group in the siRNA or siRNA conjugate in the pharmaceutical composition comprises a sense strand and an antisense strand, the sense strand comprises a nucleotide sequence I, and the antisense strand comprises a nucleotide sequence II, wherein the nucleotide sequence I and the nucleotide sequence II each consist of 19 nucleotides, each of the nucleotides in the nucleotide sequence I and the nucleotide sequence II is a modified or unmodified nucleotide, the nucleotide sequence I and the nucleotide sequence II are at least partially reverse complementary to form a double-stranded region, the nucleotide sequence II is at least partially reverse complementary to a first nucleotide sequence segment, and the first nucleotide sequence segment is a nucleotide sequence of 19 nucleotides in length in HBV mRNA.

In some embodiments, the nucleotide sequence I has the same length and no more than three nucleotide differences from the nucleotide sequence shown in SEQ ID NO: 1; and the nucleotide sequence II has the same length and no more than three nucleotide differences from the nucleotide sequence shown in SEQ ID NO: 2:
5'-CCUUGAGGCAUACUUCAAZ₁-3' (SEQ ID NO: 1);
5'-Z₂UUGAAGUAUGCCUCAAGG-3' (SEQ ID NO: 2);
wherein Z₁ is A, Z₂ is U, the nucleotide sequence I comprises a nucleotide Z₃ at a corresponding site to Z₁, the nucleotide sequence II comprises a nucleotide Z₄ at a corresponding site to Z₂, and Z₄ is the first nucleotide at the 5' terminal of the antisense strand;
or the nucleotide sequence I has the same length and no more than three nucleotide differences from the nucleotide sequence shown in SEQ ID NO: 3; and the nucleotide sequence II has the same length and no more than three nucleotide differences from the nucleotide sequence shown in SEQ ID NO: 4:
   5'-GUGUGCACUUCGCUUCACZ₅-3' (SEQ ID NO: 3);
   5'-Z₆GUGAAGCGAAGUGCACAC-3' (SEQ ID NO: 4);
   wherein Z₅ is A, Z₆ is U, the nucleotide sequence I comprises a nucleotide Z₇ at a corresponding site to Z₅, the nucleotide sequence II comprises a nucleotide Z₈ at a corresponding site to Z₆, and Z₈ is the first nucleotide at the 5' terminal of the antisense strand;
   or the nucleotide sequence I has the same length and no more than three nucleotide differences from the nucleotide sequence shown in SEQ ID NO: 5; and the nucleotide sequence II has the same length and no more than three nucleotide differences from the nucleotide sequence shown in SEQ ID NO: 6:
      5'-GGACUUCUCUCAAUUUUCZ₉-3' (SEQ ID NO: 5);
      5'-Z₁₀GAAAAUUGAGAGAAGUCC-3' (SEQ ID NO: 6);
      wherein Z₉ is U, Z₁₀ is A, the nucleotide sequence I comprises a nucleotide Z₁₁ at a corresponding site to Z₉, the nucleotide sequence II comprises a nucleotide Z₁₂ at a corresponding site to Z₁₀, and Z₁₂ is the first nucleotide at the 5' terminal of the antisense strand;
      or the nucleotide sequence I has the same length and no more than three nucleotide differences from the nucleotide sequence shown in SEQ ID NO: 7; and the nucleotide sequence II has the same length and no more than three nucleotide differences from the nucleotide sequence shown in SEQ ID NO: 8:
         5'-CUGUAGGCAUAAAUUGGUZ₁₃-3' (SEQ ID NO: 7);
         5'-Z₁₄ACCAAUUUAUGCCUACAG-3' (SEQ ID NO: 8);
         wherein Z₁₃ is A, Z₁₄ is U, the nucleotide sequence I comprises a nucleotide Z₁₅ at a corresponding site to Z₁₃, the nucleotide sequence II comprises a nucleotide Z₁₆ at a corresponding site to Z₁₄, and Z₁₆ is the first nucleotide at the 5' terminal of the antisense strand;
         or the nucleotide sequence I has the same length and no more than three nucleotide differences from the nucleotide sequence shown in SEQ ID NO: 9; and the nucleotide sequence II has the same length and no more than three nucleotide differences from the nucleotide sequence shown in SEQ ID NO: 10:
            5'-GUGCACUUCGCUUCACZ₁₇-3' (SEQ ID NO: 9);
            5'-Z₁₈ACCAAUUUAUGCCUACAG-3' (SEQ ID NO: 10);
            wherein Z₁₇ is A, Z₁₈ is U, the nucleotide sequence I comprises a nucleotide Z₁₉ at a corresponding site to Z₁₇, the nucleotide sequence II comprises a nucleotide Z₂₀ at a corresponding site to Z₁₈, and Z₂₀ is the first nucleotide at the 5' terminal of the antisense strand.

In some embodiments, the sense strand and the antisense strand are identical or different in length; the sense strand is 19-23 nucleotides in length, and the antisense strand is 20-26 nucleotides in length. Thus, the length ratio of the sense strand to the antisense strand in the siRNA or siRNA group may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the sense strand is 19 nucleotides in length, and the antisense strand is 21 nucleotides in length. In some embodiments, the sense strand is 21 nucleotides in length, and the antisense strand is 23 nucleotides in length.

In some embodiments, the nucleotide sequence I further comprises a nucleotide sequence III, and the nucleotide sequence II further comprises a nucleotide sequence IV, wherein the nucleotide sequence III and the nucleotide sequence IV are identically 1-4 nucleotides in length; the nucleotide sequence III is linked to the 5' terminal of the nucleotide sequence I, and the nucleotide sequence IV is linked to the 3' terminal of the nucleotide sequence II. In some embodiments, the base sequence of the nucleotide sequence IV is reversely complementary to a second nucleotide sequence segment, and the second nucleotide sequence segment is a nucleotide sequence segment adjacent to the 5' terminal of the first nucleotide sequence segment in HBV mRNA and having the same length as the nucleotide sequence IV.

In some embodiments, the nucleotide sequence III has the same length and is completely reverse complementary to the nucleotide sequence IV. Therefore, given the base(s) of the nucleotide sequence III, the base(s) of the nucleotide sequence IV is determined.

In some embodiments, the sense strand and the antisense strand are different in length, and the nucleotide sequence II further comprises a nucleotide sequence V. The nucleotide sequence V is 1 to 3 nucleotides in length and is linked to the 3' terminal of the antisense strand, constituting the 3' overhang of the antisense strand. Thus, the length ratio of the sense strand to the antisense strand may be 19/20, 19/21, 19/22, 20/21, 20/22, 20/23, 21/22, 21/23, 21/24, 22/23, 22/24, 22/25, 23/24, 23/25 or 23/26. In some embodiments, the nucleotide sequence V is 2 nucleotides in length, and thus, the length ratio of the sense strand to the antisense strand may be 19/21, 21/23 or 23/25.

Each nucleotide in the nucleotide sequence V may be any nucleotide. For ease of synthesis and cost saving, in some embodiments, the nucleotide sequence V is 2 consecutive thymine deoxyribonucleotides (dTdT) or 2 consecutive uracil ribonucleotides (UU); or, to improve the affinity of the antisense strand for the target mRNA, the nucleotide sequence V is complementary to a nucleotide at a corresponding site of the target mRNA. Thus, in some embodiments, the length ratio of the sense strand to the antisense strand is 19/21 or 21/23. In this case, the siRNA or siRNA conjugate has better mRNA silencing activity.

In some embodiments, each nucleotide in the siRNA group in the siRNA or siRNA conjugate in the pharmaceutical composition is a modified nucleotide. In some embodiments, each nucleotide in the nucleotide sequence I and the nucleotide sequence II is a fluoro modified or non-fluoro modified nucleotide; in the direction from 5' terminal to 3' terminal, in the sense strand, nucleotides at positions 7, 8, and 9 of the nucleotide sequence I are fluoro modified nucleotides; in the direction from 5' terminal to 3' terminal, in the antisense strand, nucleotides at positions 2, 14, and 16 of the nucleotide sequence II are fluoro modified nucleotides; each fluoro modified nucleotide is independently selected from a nucleotide formed by substituting the hydroxy at the 2' position of the ribosyl group of a nucleotide with fluoro, and each non-fluoro modified nucleotide is independently selected from a nucleotide or nucleotide analog formed by substituting the hydroxy at the 2' position of the ribosyl group of a nucleotide with a non-fluoro group. In some embodiments, in the direction from 5' terminal to 3' terminal, in the sense strand, nucleotides at positions 7, 8 and 9, or 5, 7, 8 and 9 of the nucleotide sequence I are fluoro modified nucleotides; in the direction from 5' terminal to 3' terminal, in the antisense strand, nucleotides at positions 2, 6, 14 and 16, or 2, 6, 8, 9, 14 and 16 of the nucleotide sequence II are fluoro modified nucleotides; and the other nucleotides in the sense strand and the antisense strand are non-fluoro modified nucleotides.

In the context of the present disclosure, a fluoro modified nucleotide refers to a nucleotide formed by substituting the hydroxy at the 2' position of the ribosyl group of a nucleotide with fluoro, which has the structure represented by the following formula (7). A non-fluoro modified nucleotide refers to a nucleotide or nucleotide analog formed by substituting the hydroxy at the 2' position of the ribosyl group of a nucleotide with a non-fluoro group. In some embodiments, each of the non-fluoro modified nucleotides is independently selected from one of a 2'-alkoxy modified nucleotide, a 2'-substituted alkoxy modified nucleotide, a 2'-alkyl modified nucleotide, a 2'-substituted alkyl modified nucleotide, a 2'-amino modified nucleotide, a 2'-substituted amino modified nucleotide, and a 2'-deoxynucleotide. In some embodiments, the 2'-alkoxy modified nucleotide is a methoxy modified nucleotide (2'-OMe), represented by formula (8). In some embodiments, the 2'-substituted alkoxy modified nucleotide may be, for example, a 2'-O-methoxyethyl modified nucleotide (2'-MOE), represented by formula (9). In some embodiments, the 2'-amino modified nucleotide (2'-NH₂) is represented by formula (10). In some embodiments, the 2'-deoxynucleotide (DNA) is represented by formula (11):

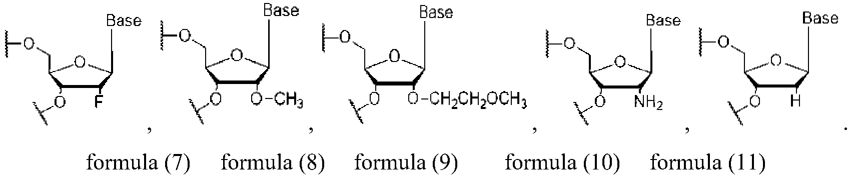

A bridged nucleic acid (BNA) refers to a constrained or inaccessible nucleotide. The BNA may contain a five-membered, six-membered, or seven-membered ring bridged structure with a "fixed" C3'-endo sugar puckering. Typically, the bridge is incorporated at the 2'- and 4'-positions of the ribose to provide a 2',4'-BNA nucleotide. In some embodiments, the BNA may be an LNA, an ENA, an cET BNA, etc., wherein the LNA is represented by formula (12), the ENA is represented by formula (13), and the cET BNA is represented by formula (14):

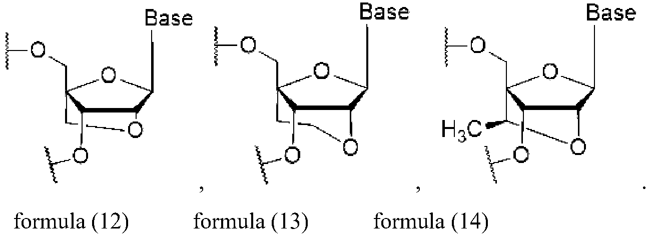

An acyclic nucleotide is a type of nucleotide formed by opening the ribose ring of the nucleotide. In some embodiments, the acyclic nucleotide may be an unlocked nucleic acid (UNA) or a glycerol nucleic acid (GNA), wherein the UNA is represented by formula (15), and the GNA is represented by formula (16):

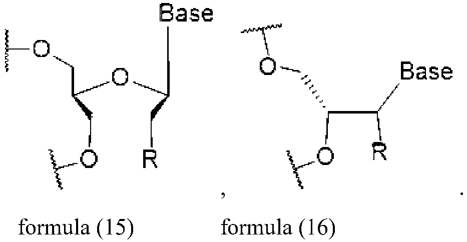

In formula (15) and formula (16), R is selected from H, OH or alkoxy (O-alkyl).

An isonucleotide refers to a compound formed by changing the position of the base in the nucleotide on the ribose ring. In some embodiments, the isonucleotide may be a compound formed by moving a base from the 1'-position to the 2'-position or 3'-position of the ribose ring, represented by formula (17) or (18).

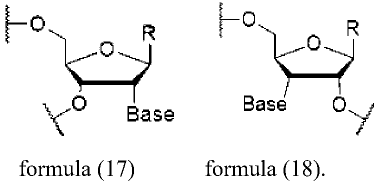

In the compounds of formulas (17)-(18), Base represents a nucleic acid base, such as A, U, G, C or T; and R is selected from H, OH, F or the non-fluoro group as described above.

In some embodiments, the nucleotide analog is selected from one of an isonucleotides, an LNA, an ENA, a cET, a UNA and a GNA. In some embodiments, each of the non-fluoro modified nucleotides is a methoxy modified nucleotide. In the text above and below, the methoxy modified nucleotide refers to a nucleotide formed by substituting the 2'-hydroxy of the ribosyl group with a methoxy group.

In the text above and below, "fluoro modified nucleotide", "2'-fluoro modified nucleotide", "nucleotide in which the 2'-hydroxy of the ribosyl group is substituted with fluoro" and "nucleotide with 2'-fluororibosyl group" have the same meaning, referring to a compound which is formed by substituting the 2'-hydroxy of the nucleotide with fluoro and having a structure represented by formula (7); "methoxy modified nucleotide", "2'-methoxy modified nucleotide", "nucleotide in which the 2'-hydroxy of the ribosyl group is substituted with methoxy" and "nucleotide with 2'-methoxyribosyl group" have the same meaning, referring to a compound which is formed by substituting the 2'-hydroxy of the ribosyl group of the nucleotide with methoxy and having a structure represented by formula (8).

In some embodiments, in the pharmaceutical composition, at least 1 phosphate group in a phosphate-ribose backbone of at least one single strand of the sense strand and the antisense strand is a phosphate group with a modified group. In some embodiments, the phosphate group with the modified group is a phosphorothioate group formed by substituting at least one oxygen atom in a phosphodiester bond in the phosphate group with a sulfur atom. In some embodiments, the phosphate group with the modified group is a phosphorothioate group with a structure represented by formula (1):

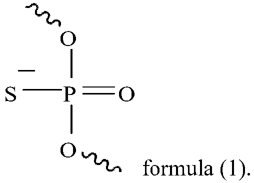

In some embodiments, the phosphorothioate group linkage exists in at least one of the following positions: the position between the first and second nucleotides at either end of the sense strand or the antisense strand; the position between the second and third nucleotides at either end of the sense strand or the antisense strand; or any combination of the above. In some embodiments, the phosphorothioate group linkage exists in all of the above positions except 5' terminal of the sense strand. In some embodiments, the phosphorothioate group linkage exists in all of the above positions except 3' terminal of the sense strand. In some embodiments, the phosphorothioate group linkage exists in at least one of the following positions:
the position between the first nucleotide and the second nucleotide at 5' terminal of the sense strand;
the position between the second nucleotide and the third nucleotide at 5' terminal of the sense strand;
the position between the first nucleotide and the second nucleotide at 3' terminal of the sense strand;
the position between the second nucleotide and the third nucleotide at 3' terminal of the sense strand;
the position between the first nucleotide and the second nucleotide at 5' terminal of the antisense strand;
the position between the second nucleotide and the third nucleotide at 5' terminal of the antisense strand;
the position between the first nucleotide and the second nucleotide at 3' terminal of the antisense strand; and
the position between the second nucleotide and the third nucleotide at 3' terminal of the antisense strand.

In some embodiments, in the pharmaceutical composition of the present disclosure, a 5' terminal nucleotide of the antisense strand is a 5'-phosphate nucleotide or a 5'-phosphate analog modified nucleotide. Commonly used 5'-phosphate nucleotides or 5'-phosphate analog modified nucleotides are well known to those skilled in the art. For example, the 5'-phosphate nucleotides may have the structure represented by formula (2):

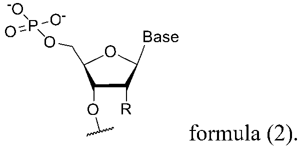

In another example, Anastasia Khvorova and Jonathan K. Watts, The chemical evolution of oligonucleotide therapies of clinical utility. Nature Biotechnology, 2017, 35(3): 238-48 discloses the following four 5'-phosphate analog modified nucleotides: 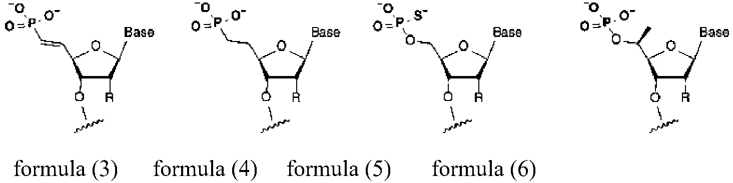 wherein R is selected from H, OH, methoxy, and fluoro; and Base represents a base selected from A, U, C, G and T.

In some embodiments, the 5'-phosphate nucleotide is a nucleotide containing a 5'-phosphate modification represented by formula (2), and the 5'-phosphate analog modified nucleotide is a nucleotide containing 5'-(E)-vinylphosphonate (E-VP) modification represented by formula (3), or phosphorothioate modified nucleotide represented by formula (5).

In some embodiments, the sense strand in the siRNA is the nucleotide sequence shown in SEQ ID NO: 11, and the antisense strand is the nucleotide sequence shown in SEQ ID NO: 12:
5'-CmsCmsUmUmGmAmGfGfCfAmUmAmCmUmUmCmAmAmAm-3'
   (SEQ ID NO: 11);
5'-VP-UmsUfsUmGmAmAfGmUmAmUmGmCmCmUfCmAfAmGmGmsUmsUm-3'
   (SEQ ID NO: 12);
or the sense strand in the siRNA is the nucleotide sequence shown in SEQ ID NO: 13, and the antisense strand is the nucleotide sequence shown in SEQ ID NO: 14:
5'-GmsUmsGmUmGfCmAfCfUfUmCmGmCmUmUmCmAmCmAm-3'
   (SEQ ID NO: 13);
5'-UmsGfsUmGmAm(Agn)GmCfGfAmAmGmUmGfCmAfCmAmCmsUmsUm-3'
   (SEQ ID NO: 14);
or the sense strand in the siRNA is the nucleotide sequence shown in SEQ ID NO: 15, and the antisense strand is the nucleotide sequence shown in SEQ ID NO: 16:
5'-(invAb)sGmUmGmGmAmCmUmUmCfUfCfUmCmAmAmUmUmUmUmCmUms(invAb)-3'
   (SEQ ID NO: 15);
5'-AmsGfsAmsAfAmAfUmUfGmAfGmAfGmAfAmGfUmCfCmAmsCm-3'
   (SEQ ID NO: 16);
or the sense strand in the siRNA is the nucleotide sequence shown in SEQ ID NO: 17, and the antisense strand is the nucleotide sequence shown in SEQ ID NO: 18:
5'-(invAb)sCmGmCmUmGmUmAmGmGfCfAfUmAmAmAmUmUmGmGmUmAms(invAb)-3'
   (SEQ ID NO: 17);
5'-UmsAfsCmsCfAmAfUmUfUmAfUmGfCmCfUmAfCmAfGmCmsGm-3'
   (SEQ ID NO: 18);
or the sense strand in the siRNA is the nucleotide sequence shown in SEQ ID NO: 19, and the antisense strand is the nucleotide sequence shown in SEQ ID NO: 20:
5'-GmsUmsGmCmAfCfUfUmCmGmCmUmUmCmAmCmAm-3' (SEQ ID NO: 19);
5'-UmsGfsUmGmAmAmGmCmGmAmAmGmUmGfCmAfCmAmCmsGmsGmUf-3' (SEQ ID NO: 20);
or the sense strand in the siRNA is the nucleotide sequence shown in SEQ ID NO: 21, and the antisense strand is the nucleotide sequence shown in SEQ ID NO: 22:
5'-GmsUmsGmCmAfCfUfUmCmGmCmUmUmCmAmCmAm-3' (SEQ ID NO: 21);
5'-UmsGfsUmGm(Agn)AmGmCmGmAmAmGmUmGfCmAfCmAmCmsGmsGmUf-3' (SEQ ID NO: 22);
or the sense strand in the siRNA is the nucleotide sequence shown in SEQ ID NO: 23, and the antisense strand is the nucleotide sequence shown in SEQ ID NO: 24:
5'-GmsUmsGmCmAfCfUfUmCmGmCmUmUmCmAmCmAm-3' (SEQ ID NO: 23);
5'-UmsGfsUmGmAm(Agn)GmCmGmAmAmGmUmGfCmAfCmAmCmsGmsGmUf-3' (SEQ ID NO: 24).

The uppercase letters C, G, U, and A represent the base composition of nucleotides; the lowercase letter m indicates that the nucleotide adjacent to the letter m on the left side is a methoxy modified nucleotide; the lowercase letter f indicates that the nucleotide adjacent to the letter f on the left side is a fluoro modified nucleotide; the lowercase letter s indicates that the two nucleotides on the left and right sides of the letter are linked by a phosphorothioate group; the letter combination VP indicates that the nucleotide adjacent to the letter combination VP on the right side is a 5'-(E)-vinylphosphonate (E-VP) modified nucleotide. invAb represents inverted abasic deoxyribonucleotide; Agn represents adenosine glycerol nucleic acid (GNA).

### Immune response regulator

"Immune response regulator" refers to an agent that can regulate an immune response, and participates in the regulation of the immune response by promoting or inhibiting the mechanisms and effects of cellular immunity and humoral immunity. In some embodiments, the immune response regulator includes, but is not limited to, an adjuvant or an immune response stimulant. In some embodiments, the adjuvant is selected from one or more of agents capable of promoting an immune response. In some embodiments, the immunostimulant is selected from one or more of independently administrable reagents capable of stimulating an immune response.

As used herein, the term "adjuvant" or "vaccine adjuvant" is to be understood as a reagent that promotes (e.g., enhances, accelerates, or prolongs) an immune response to an antigen with which it is administered to elicit a long-term protective immunity. There is no substantial immune response against the adjuvant itself. Adjuvants include, but are not limited to, pathogen components, particulate adjuvants, and combined adjuvants (see, e.g., www.niaid.nih.gov/research/vaccine-adjuvants-types). Pathogen components (e.g., monophosphoryl lipid A (MPL), poly(I:C), poly ICLC adjuvant, CpG DNA, c-di-AMP, c-di-GMP, and c-di-CMP; short and blunt-ended 5'-triphosphate dsRNA (3pRNA) RIG-1 ligand, and emulsion, such as poly[di(carboxyethylphenoxysodium)phosphazene] (PCEP)) can help trigger an early non-specific or innate immune response to a vaccine by targeting various receptors inside or on the surface of innate immune cells. The innate immune system affects the acquired immune response, which provides long-term protection against vaccine-targeted pathogens. Particulate adjuvants (e.g., alum, virosomes, and cytokines, such as IL-12) form very small particles, which can stimulate the immune system, and can also enhance delivery of antigens to immune cells. Combined adjuvants, such as AS02, AS03 and AS04 (all GSK); MF59 (Novartis); (CSL Limited); and (Altimmune), elicit a variety of protective immune responses. Adjuvants that are moderately effective when used alone can induce a more potent immune response when used together.

In some embodiments, the adjuvant used in the present disclosure promotes a humoral immune response and a cellular immune response. To this end, a balanced Th1/Th2 helper T cell response is required to support neutralizing antibody responses as well as effector cell cytotoxic T cell responses. In some embodiments, the adjuvant provides a balanced Th1/Th2 response. In certain embodiments, the adjuvant is one or more of poly I:C adjuvant, poly ICLC adjuvant, CpG adjuvant, STING agonist (c-di-AMP adjuvant, c-di-GMP adjuvant or c-di-CMP adjuvant), ISCOMATRIX^{®} adjuvant, PCEP adjuvant and Rig-I-ligand adjuvant. In some embodiments, the adjuvant is poly I:C adjuvant, CpG adjuvant, STING agonist, or PCEP adjuvant. In some embodiments, the adjuvant is CpG adjuvant.

As used herein, an "immunostimulant" is a reagent that stimulates an immune response, which may or may not be administered independently of the antigen. Immunostimulants include, but are not limited to, pegylated interferon α2a (PEG-IFN-α-2a), interferon α-2b, PEG-IFN α-2b, interferon λ, recombinant human interleukin 7 and Toll-like receptor 3, 7, 8 or 9 (TLR3, TLR7, TLR8, or TLR9) agonists, viral entry inhibitors (e.g., Myrcludex), oligonucleotides that inhibit HBsAg secretion or release (e.g., REP 9AC), capsid inhibitors (e.g., Bay41-4109 and NVR-1221), cccDNA inhibitors (e.g., IHVR-25). In some embodiments, the immunostimulant may comprise a viral capsid, optionally an empty viral capsid, e.g., an MVA capsid. In some embodiments, the immunostimulant may also include an immune checkpoint regulator. The immune checkpoint regulator may be stimulatory or inhibitory. As used herein, immune checkpoint regulators enhance the immune response. Immune checkpoint regulators include, but are not limited to, CTLA-4 inhibitors (e.g., ipilimumab) and PD-1 inhibitors (e.g., nivolumab, pembrolizumab, and BGB-A317 antibody). In addition to affimer biotherapeutics, PD-L1 inhibitors include atezolizumab, avelumab, and durvalumab.

In some embodiments, the immune response regulator in the pharmaceutical composition of the present disclosure is selected from one or more of TLR agonists. In some embodiments, the immune response regulator is selected from one or more of TLR9 agonists. In some embodiments, the immune response regulator is selected from one or more of CpG DNA or a pharmaceutically acceptable salt thereof and an alum adjuvant. In some embodiments, the CpG DNA comprises the nucleotide sequence shown in SEQ ID NO 25 or SEQ ID NO 26:
5'-TCGTCGTTTTGTCGTTTTGTCGTT-3' (SEQ ID NO 25)
5'-TGACTGTGAACGTTCGAGATGA-3' (SEQ ID NO 26);
wherein each nucleotide in the CpG DNA is a deoxynucleotide, and the nucleotides in the CpG DNA are linked by a phosphorothioate ester bond. In some embodiments, the CpG DNA is a commercial adjuvant CpG 7909 or CpG 1018.

In some embodiments, the composition of the present disclosure comprises an RNAi agent and one or more immune response regulators, or comprises an RNAi agent and one or more adjuvants, or comprises an RNAi agent and one or more of a particulate adjuvant and a pathogen component, or comprises an RNAi agent and CpG DNA and/or an alum adjuvant. In some embodiments, the pharmaceutically active component of the composition of the present disclosure comprises only an RNAi agent and CpG DNA. In some embodiments, the pharmaceutically active component of the composition of the present disclosure comprises an RNAi agent and CpG DNA and/or an alum adjuvant. In some embodiments, CpG DNA and the alum adjuvant may be administered simultaneously or non-simultaneously.

In some embodiments, in the composition of the present disclosure, the RNAi agent is a conjugate having a structure represented by formula (403), a sodium salt thereof, or a partial sodium salt thereof, wherein Nu represents an siRNA group, the siRNA group has a sense strand shown in SEQ ID NO: 11 and an antisense strand shown in SEQ ID NO: 12, and the siRNA group is formed by removing one hydrogen atom from the 3' hydroxy of the 3' terminal nucleotide of the sense strand; the immune response regulator is a CpG DNA shown in SEQ ID NO: 25, a sodium salt thereof, or a partial sodium salt thereof.

The siRNA, the siRNA conjugate and the immune response regulator described above can be readily prepared by those skilled in the art through suitable prior art methods, or are commercially available. For example, WO2015006740A2 describes preparation methods for various siRNA conjugates in detail. A method for preparing the structure represented by formula (305) is described in WO2014025805A1. The preparation method for the structure represented by formula (307) is described by Rajeev et al. in ChemBioChem 2015, 16, 903-908. Chinese patent application CN110959011A also discloses in detail a method for preparing the siRNA conjugate represented by formula (308). The contents of the documents are incorporated herein in their entirety by reference. For another example, siRNA and CpG DNA can be obtained by nucleic acid solid phase synthesis methods well known in the art by linking nucleoside phosphoramidite monomers one by one in the base order of the siRNA nucleic acid sequence.

In some embodiments, a pharmaceutically acceptable salt of one or more of the siRNA, the siRNA conjugate, and the CpG DNA is a water-soluble salt or partial salt. In some embodiments, the pharmaceutically acceptable salt is an alkali metal salt or a partial alkali metal salt. In some embodiments, the pharmaceutically acceptable salt is a sodium salt or partial sodium salt. In some embodiments, the RNAi agent is a sodium salt of the siRNA conjugate, and the immune response regulator is a sodium salt of CpG DNA.

In some embodiments, to facilitate transportation and/or storage, the siRNA and the siRNA conjugate in the RNAi agent and the immune response regulator are each independently present in the form of a powder, e.g., in the form of a lyophilized powder for injection. During administration, the lyophilized powder for injection is mixed with a liquid excipient to prepare a liquid agent. In some embodiments, for ease of use, the siRNA and the siRNA conjugate in the RNAi agent and the immune response regulator are present in various RNAi formulations and immune response regulator formulations commonly used in the art. For example, the RNAi agent and the immune response regulator formulation may each independently be a liquid agent, e.g., an injection solution. The liquid agent may be an injection solution for subcutaneous injection, an injection solution for intraperitoneal injection, an injection solution for intramuscular injection, or an injection solution for intravenous injection, or may be a spray administered to the lungs by spraying or administered to other organs (e.g., the liver) through the lungs by spraying, or an inhalant inhaled through the oropharynx, or a pharmaceutical agent administered through the nasal cavity. Therefore, the RNAi agent comprises at least one of the siRNA and the siRNA conjugate and a pharmaceutically acceptable carrier and/or adjuvant, and the types and contents of the carriers and/or the adjuvants in the injection solution for subcutaneous injection, the injection solution for intramuscular injection, the injection solution for intravenous injection, the spray administered to the lungs by spraying or administered to other organ tissues (e.g., the liver) through the lungs by spraying, the inhalant inhaled through the oropharynx, or the pharmaceutical agent administered through the nasal cavity are well known to those skilled in the art. In some embodiments, the RNAi agent is an injection solution for subcutaneous injection.

### Auxiliary agent

The pharmaceutical composition also comprises an auxiliary agent selected from one or more of a solvent and a pharmaceutically acceptable carrier.

When the pharmaceutical composition is an injection solution, the auxiliary agent at least comprises a solvent. The solvent may be, for example, deionized water, water for injection, ethanol, or a pH buffer solution. The pH buffer solution may be a Tris hydrochloride buffer with a pH value of 7.5-8.5 and/or a phosphate buffer solution with a pH value of 5.5-8.5, for example, a phosphate buffer solution with a pH value of 5.5-8.5.

The amount of the solvent is adjusted according to the required concentration of the solution. The concentration of the conjugate in the injection solution may be 0.01 mg/mL to 20 mg/mL, 0.1 mg/mL to 10 mg/mL, or 0.5 mg/mL to 5 mg/mL, based on the nucleotide sequence group in the conjugate.

The pharmaceutically acceptable carrier is one or more of various components conventionally used in the art, such as one or more of a protective agent, an osmotic pressure regulator and other pharmaceutically acceptable carriers.

The other pharmaceutically acceptable carrier may be a carrier commonly used in the field, such as but not limited to magnetic nanoparticles (such as nanoparticles based on Fe₃O₄ or Fe₂O₃), carbon nanotubes, mesoporous silicon, calcium phosphate nanoparticles, polyethyleneimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid)copolymer (PLGA), poly(2-aminoethyl ethylene phosphate) (PPEEA) and poly(2-dimethylaminoethyl methacrylate) (PDMAEMA) as well as derivatives thereof.

In some embodiments, the pharmaceutically acceptable carrier comprises a physiologically acceptable compound that functions, for example, to stabilize the pharmaceutical composition or to increase or decrease the absorption of the conjugate and/or the pharmaceutical composition. The physiologically acceptable compound is selected from one or more of the following compounds: carbohydrates, such as glucose, sucrose and/or dextran; antioxidants, such as ascorbic acid and/or glutathione; low molecular weight proteins; compositions that reduce the clearance or hydrolysis of any co-administered substance; excipients; stabilizers and buffer agents. Detergents may also be used to stabilize the composition or to increase or decrease the absorption of the pharmaceutical composition. The physiologically acceptable compound may also comprise one or more of a wetting agent, an emulsifying agent, a dispersing agent, or a preservative that is specifically used to prevent the growth or action of microorganisms. The physiologically acceptable compound is known to those skilled in the art and will not be described in the present disclosure. Those skilled in the art will readily appreciate that the selection of a pharmaceutically acceptable carrier and a physiologically acceptable compound depends, for example, on the route of administration and the particular physiochemical properties of any co-administered substance.

In some embodiments, the pharmaceutically acceptable carrier is sterile and generally free of undesirable substances. The pharmaceutical composition provided by the present disclosure may further comprise a pharmaceutically acceptable auxiliary substance as needed to approximate physiological conditions, such as pH regulators and buffer agents, toxicity regulators, etc., e.g., sodium acetate, sodium chloride, potassium chloride, calcium chloride, sodium lactate, etc. The concentration of the drug conjugate provided by the present disclosure in the pharmaceutical composition may vary within a wide range and is selected primarily according to fluid volume, viscosity, body weight, etc., and a particular mode of administration.

In some embodiments, in the pharmaceutical composition, there is no special requirement on the content of the pharmaceutically active ingredient and the pharmaceutically acceptable carrier. In some embodiments, in the pharmaceutically active ingredient, based on siRNA in the RNAi agent, the weight ratio of the RNAi agent to the auxiliary agent may be 1:(1-600), and in some embodiments, the weight ratio described above is 1:(1-50). In some embodiments, in the pharmaceutically active ingredient, the weight ratio of the immune response regulator to the auxiliary agent may be 1:(1-5000), and in some embodiments, the weight ratio described above is 1:(1-500).

In some embodiments, the carrier may be selected from an osmotic pressure regulator, which may be sodium chloride and/or potassium chloride. The content of the osmotic pressure regulator makes the osmotic pressure of the pharmaceutical composition 200-700 milliosmol/kg (mOsm/kg). According to the desired osmotic pressure, those skilled in the art may easily determine the content of the osmotic pressure regulator. In some embodiments, the dose of the agent made from the pharmaceutical composition will be adjusted due to different administration methods during administration.

In some embodiments, the carrier may be selected from a protective agent. The protective agent may be at least one of inositol, sorbitol, sucrose, trehalose, mannose, maltose, lactose and glucose. Based on the total weight of the pharmaceutical composition, the content of the protective agent may be 0.01-30% by weight.

In some embodiments, the pharmaceutical composition may be a liquid agent, such as an injection solution; it may also be a freeze-dried powder injection, which is mixed with a liquid excipient during administration to prepare a liquid agent. The liquid agent may be used for, but is not limited to, subcutaneous, intramuscular, or intravenous injection, and may also be used for, but is not limited to, delivering the pharmaceutical composition by puncture injection, or by oropharyngeal inhalation, or by nasal administration, etc. In some embodiments, the pharmaceutical composition is used for subcutaneous, intramuscular, intravenous, or intrathecal injection administration. In some embodiments, the RNAi agent is present in a formulation for subcutaneous injection, and the immune response regulator is present in a formulation for intraperitoneal injection or subcutaneous injection.

### Use and treatment method of the pharmaceutical composition of the present disclosure

In another aspect, the present disclosure provides use of the pharmaceutical composition of the present disclosure in preparing a medicament for treating a disease associated with hepatitis B virus infection. In some embodiments, the disease associated with hepatitis B virus infection is one or more of inflammation caused by hepatitis B virus infection, hepatic fibrosis, liver proliferative disease, liver failure, and hepatocellular carcinoma. In some embodiments, the inflammation caused by hepatitis B virus infection refers to hepatitis B and/or hepatitis D.

In yet another aspect, the present disclosure also provides a method for treating a disease associated with hepatitis B virus infection, comprising administering to a subject in need an effective amount of the pharmaceutical composition of the present disclosure. In some embodiments, the method comprises administering to a subject in need an effective amount of an RNAi agent and an effective amount of an immune response regulator, wherein the RNAi agent can inhibit HBV-expressed mRNA. In some embodiments, the disease associated with hepatitis B virus infection is selected from one or more of the group consisting of the following diseases: hepatitis, liver fibrosis, and liver proliferative disease.

The pharmaceutical composition of the present disclosure can significantly reduce the levels of HBV antigens such as HBsAg and HBeAg, and HBV DNA in a subject. Further, the pharmaceutical composition of the present disclosure can also induce the production of HBV antibody in a subject, showing excellent potential for functional cure. In the context of the present disclosure, when the HBV antigens, the content of DNA and the content of HBV antibody are calculated using logarithmic coordinates, the unit of measurement used is log10 (IU/mL or S/CO), which means a logarithmic content value obtained by performing common logarithmic calculation on the concentration content (IU/mL or S/CO). For example, the initial content is 5 log₁₀ (IU/mL) and is reduced to 4 log₁₀ (IU/mL), and the concentration is reduced by 1 log₁₀ (IU/mL), which means that the concentration is reduced from 10⁵ IU/mL to 10⁴ IU/mL, i.e., to 1/10 of the initial concentration.

In some embodiments, the treatment method of the present disclosure comprises administering to a subject in need the RNAi agent and the anti-hepatitis B virus antibody in 1 or more cycles. In some embodiments, the treatment method of the present disclosure comprises 1-4 of the cycles described above. In some embodiments, the cycle is 5-120 weeks in length. In some embodiments, the cycle is 5-96 weeks in length. In some embodiments, the cycle is 7 weeks, 50 days, 60 days, 70 days, 80 days, 12 weeks, 20 weeks, 24 weeks, 36 weeks, 48 weeks, or 60 weeks in length. In some embodiments, 1 of the cycles described above is referred to as 1 complete "treatment course".

It will be understood by those skilled in the art that after the functional cure of HBV in a subject is achieved, there is generally no need to continue the treatment. Thus, in some embodiments, the treatment method of the present disclosure does not require the completion of the last complete treatment course, and the administration is stopped when the HBsAg concentration in the serum of the subject is below the lower limit of detection of the kit, i.e., 0.05 IU/mL. In some embodiments, the administration may be stopped when the absolute value of HBsAg in the serum of the subject is 100 IU/mL or less.

In some embodiments, the RNAi agent and the immune response regulator are administered separately for better efficacy of the prepared drug. In some embodiments, the RNAi agent is administered to the subject before the immune response regulator is administered. In some embodiments, the immune response regulator is administered to the subject before the RNAi agent is administered. In some embodiments, the time interval between the first administration of the RNAi agent and the first administration of the immune response regulator is 0 days to one month, e.g., 0 days, 1 day, 2 days, 3 days, 5 days, 1 week, 2 weeks, 3 weeks, or 1 month. In some embodiments, the interval of the first administrations of the RNAi agent and the immune response regulator is 2 weeks.

In some embodiments, the method comprises one or more treatment courses, wherein the RNAi agent and the immune response regulator are each independently administered one or more times in one course of treatment. In some embodiments, in one treatment course, an effective amount of the RNAi agent is administered to the subject before the immune response regulator is administered. In some embodiments, in one treatment course, after 0.01-27 mg/kg of the RNAi agent is administered to the subject, 0.05-3 mg of the immune response regulator is first administered.

In some embodiments, within one treatment course, the RNAi agent is administered once or more times, and the immune response regulator is administered once or more times. In some embodiments, the RNAi agent is administered 1-5 times, and the immune response regulator is administered 1-6 times. In some embodiments, to obtain a better therapeutic effect, the RNAi agent is administered 1-3 times, and the immune response regulator is administered 2-4 times.

In some embodiments, to reduce the total dose and frequency of administration, the interval between the times of each administration of the RNAi agent is 5 days to 60 weeks, 7 days to 60 weeks, or 7 days to 1 year. In some embodiments, in order to obtain a better therapeutic effect, specifically, in some embodiments, the RNAi agent is administered at an interval of 7 days, 14 days, 21 days, 1 month, 2 months, 3 months, half a year, or 1 year between the times of each administration.

In some embodiments, the interval between the times of each administration of the immune response regulator is 1 day to 2 months, 2 days to 1 month, 3 days to 1 month, 1 week to 1 month, or 2 weeks to 1 month. Specifically, in some embodiments, the interval between the times of each administration of the immune response regulator is 3 days, 5 days, 6 days, 1 week, 2 weeks, 3 weeks, or 1 month.

In some embodiments, in the process of preparing a drug, the RNAi agent and the immune response regulator are present in a form suitable for administration to a subject once or more times within 1 treatment course, the length of the treatment course being 25-840 days. In some embodiments, the treatment course is 25-672 days, 25-504 days, 25-420 days, 25-336 days, 25-252 days, 25-168 days, 70-504 days, 70-336 days, and 70-168 days in length.

In some embodiments, within one of the treatment courses, the RNAi agent is administered once or more times, and the immune response regulator is administered once or more times. In some embodiments, the RNAi agent is administered 1-5 times, and the immune response regulator is administered 2-6 times. In some embodiments, to obtain a better therapeutic effect, the RNAi agent is administered 1-3 times, and the immune response regulator is administered 3-4 times.

In some embodiments, to obtain a more desirable therapeutic effect over time, each administration of the RNAi agent is performed at an interval of 5 days to 60 weeks, 7 days to 60 weeks, 7 days to 1 year, or 10 days to 40 weeks. In some embodiments, in order to obtain a better therapeutic effect, specifically, in some embodiments, the RNAi agent is administered at an interval of 7 days, 14 days, 21 days, 1 month, 2 months, 3 months, half a year, or 1 year between the times of each administration.

In some embodiments, the interval between the times of each administration of the immune response regulator is 1 day to 2 months, 2 days to 1 month, 3 days to 1 month, 1 week to 1 month, and 2 weeks to 1 month. Specifically, in some embodiments, the interval between the times of each administration of the immune response regulator is 3 days, 5 days, 6 days, 1 week, 2 weeks, 3 weeks, or 1 month.

In some embodiments, based on the total amount of the siRNA and the siRNA group, the RNAi agent is administered at a single dose of 0.1 mg/kg-10 mg/kg, 0.2 mg/kg-8 mg/kg, 0.3 mg/kg-9 mg/kg, and 0.5 mg/kg-6 mg/kg body weight of the subject. Specifically, in some embodiments, the RNAi agent is administered at a single dose of 0.1 mg/kg, 0.2 mg/kg, 0.25 mg/kg, 0.3 mg/kg, 0.4 mg/kg, 0.5 mg/kg, 0.6 mg/kg, 0.8 mg/kg, 1 mg/kg, 2 mg/kg, 3 mg/kg, 4 mg/kg, 6 mg/kg, 8 mg/kg, 9 mg/kg, or 10 mg/kg body weight of the subject.

In some embodiments, based on the amount of the immune response regulator, the immune response regulator is administered at a single dose of 0.03-3 mg. In some embodiments, the immune response regulator is administered at a single dose of 0.05-2 mg, 0.1-2 mg, or 0.12-1 mg. Specifically, in some embodiments, the immune response regulator is administered at a single dose of 0.05 mg, 0.075 mg, 0.1 mg, 0.125 mg, 0.15 mg, 0.2 mg, 0.5 mg, or 0.8 mg.

In some embodiments, both the RNAi agent and the immune response regulator are administered multiple times. In some embodiments, the interval between each administration of the RNAi agent is 5 days to 60 weeks, and the interval between each administration of the immune response regulator is 1 day to 60 days. In some embodiments, the interval between each administration of the RNAi agent is 10 days to 40 weeks, and the interval between each administration of the immune response regulator is 5 days to 45 days.

In some embodiments, the immune response regulator is administered after administration of an effective amount of the RNAi agent, wherein the RNAi agent and the immune response regulator are administered at an interval of 5 days to 2 months. In some embodiments, the RNAi agent and the immune response regulator are administered at an interval of 7-45 days. In some embodiments, 2 weeks after the first administration of the RNAi agent, the immune response regulator is administered for the first time.

In some embodiments, in one treatment course, the RNAi agent is administered to the subject 1-5 times, each time at 0.1-9 mg/kg body weight of the subject, followed by 1-5 times of administration of the immune response regulator, each time at 0.05-1 mg.

In some embodiments, both the RNAi agent and the immune response regulator are present in a formulation for subcutaneous injection. In some embodiments, the RNAi agent is present in a formulation for subcutaneous injection, and the immune response regulator is present in a formulation for intraperitoneal injection.

In some embodiments, the administration of the immune response regulator may be an administration of one or more immune response regulators, or an administration of one or more adjuvants, or an administration of an aluminum hydroxide adjuvant and/or CPG DNA. In some embodiments, the single administration of the immune response regulator is a single administration of CpG DNA. In some embodiments, the single administration of the immune response regulator is a separate single administration of an alum adjuvant and CpG DNA. In the case of a separate single administration of an alum adjuvant and CpG DNA, the dose, interval and number of single administrations of the immune response regulator are calculated according to the dose, interval and number of administrations of one of the adjuvants. In some embodiments, the dose, interval, and number of administrations of the alum adjuvant and CpG DNA may be identical or different.

In some embodiments, the RNAi agent and the immune response regulator are present in a form suitable for administration to the subject during a treatment progression, wherein the treatment progression comprises one or more of the treatment courses, and the number of treatment courses can be determined according to related indexes such as hepatitis B surface antigen and HBV DNA. For example, HBsAg can be continuously reduced to 300 IU/mL or less. In some embodiments, the treatment progression comprises 1-4 of the treatment courses. In some embodiments, it will be understood by those skilled in the art that after the functional cure of HBV in a subject is achieved, there is generally no need to continue the treatment. The functional cure of HBV refers to a state in which the hepatitis B surface antigen (HBsAg) is negative (with or without the presence of a hepatitis B surface antibody), HBV DNA is undetectable, and liver function indexes are normal. Thus, in some embodiments, there is no need to complete the last complete treatment course, and the administration is stopped when the HBsAg concentration in the serum of the subject is below the lower limit of detection of the kit, i.e., 0.05 IU/mL. In some embodiments, the administration may be stopped when the absolute value of HBsAg in the serum of the subject is 100 IU/mL or less.

The definition and selection scope of the RNAi agent and the immune response regulator are as described in the previous description of the pharmaceutical composition. In some embodiments, the RNAi agent is a sodium salt of the siRNA conjugate, the siRNA group contained in the siRNA conjugate comprises the nucleotide sequences shown in SEQ ID NOs: 11 and 12, and the immune response regulator is a sodium salt of CpG 7909 comprising the sequence shown in SEQ ID NO: 25.

### Kit

In yet another aspect, the present disclosure also provides a kit comprising the pharmaceutical composition provided by the present disclosure.

In some embodiments, the kit described in the present disclosure may provide the pharmaceutical composition in one container. In some embodiments, the kit described in the present disclosure may comprise a container providing a pharmaceutically acceptable excipient. In some embodiments, the kit may further comprise other components, such as stabilizers or preservatives. In some embodiments, the kit described in the present disclosure may comprise at least one additional therapeutic agent in a container other than the container in which the pharmaceutical composition described in the present disclosure is provided. In some embodiments, the kit may comprise an instruction for mixing a pharmaceutically active ingredient in the pharmaceutical composition with a pharmaceutically acceptable carrier and/or adjuvant or other ingredients (if any).

In the kit of the present disclosure, the pharmaceutical composition, the pharmaceutically active ingredient in the pharmaceutical composition and/or the pharmaceutically acceptable adjuvant may be provided in any form, e.g., in a liquid form, a dried form or a lyophilized form. In some embodiments, the pharmaceutical composition, the pharmaceutically active ingredient in the pharmaceutical composition, and optionally the pharmaceutically acceptable adjuvant are substantially pure and/or sterile. In some embodiments, sterile water may be provided in the kit of the present disclosure.

The following examples will further illustrate the present disclosure, but the present disclosure is not limited thereby.

### EXAMPLES

Unless otherwise specified, the reagents and culture media used in the following examples are all commercially available, and the procedures used such as nucleic acid electrophoresis and real-time PCR are all performed according to the methods described in Molecular Cloning (Cold Spring Harbor Laboratory Press (1989)).

For the conjugates and CpG DNA synthesized and used in the following examples, unless otherwise specified, the sodium salts of the conjugate in which the hydroxy hydrogen ions in all phosphate groups in the conjugate are substituted with sodium ions and the sodium salts of CpG DNA in which the hydroxy hydrogen ions in all phosphate groups in CpG DNA are substituted with sodium ions were obtained.

### Preparation Example 1. Preparation of pharmaceutical compositions provided by the present disclosure

### (1-1) Preparation of RNAi agent in pharmaceutical compositions of the present disclosure

According to the preparation method described in Preparation Example 13 of CN110959011A, conjugate 1 was prepared. The sense strand and the antisense strand contained in conjugate 1 were the sequences shown in SEQ ID NO: 11 and SEQ ID NO: 12, respectively. The sense strand and the antisense strand were separately synthesized. Ultrapure water (Milli-Q ultrapure water instrument, resistivity 18.2 MΩ*cm (25 °C)) was used to dilute conjugate 1 to a concentration of 0.2 mg/mL (on a basis of siRNA group amount), and then a liquid chromatography-mass spectrometry instrument (LC-MS, purchased from Waters Inc., model: LCT Premier) was used for molecular weight determination. The theoretical molecular weight of the sense strand of conjugate 1 was 8218.83, and the measured molecular weight was 8218. The theoretical molecular weight of the antisense strand was 7061.57, and the measured molecular weight was 7061.5. The measured value was consistent with the called value, indicating that the synthesized conjugate 1 was the designed double-stranded nucleic acid sequence of interest.

Conjugate 1 has the structure represented by formula (403), and the siRNA group contained in conjugate 1 has the sequences shown in SEQ ID NO: 11 and SEQ ID NO: 12:
5'-CmsCmsUmUmGmAmGfGfCfAmUmAmCmUmUmCmAmAmAm-3' (SEQ ID NO: 11);
5'-VPUmsUfsUmGmAmAfGmUmAmUmGmCmCmUfCmAfAmGmGmsUmsUm-3' (SEQ ID NO: 12);

The uppercase letters C, G, U, and A represent the base composition of nucleotides; the lowercase letter m indicates that the nucleotide adjacent to the letter m on the left side is a methoxy modified nucleotide; the lowercase letter f indicates that the nucleotide adjacent to the letter f on the left side is a fluoro modified nucleotide; the lowercase letter s indicates that the two nucleotides on the left and right sides of the letter s are linked by a phosphorothioate group, and VP indicates that one nucleotide on the right side of the letters VP is a 5'-(E)-vinylphosphonate modified nucleotide.
(1-1-1) Preparation of component I using conjugate 1: The prepared conjugate 1 was stored at 2-8 °C in the dark, and conjugate 1 was diluted with a phosphate buffer solution to obtain a 1.8 mg/mL solution (based on the siRNA group), which was designated as component I. Component I was administered at a concentration of 1.8 mg/mL according to a dose of 5 mL/kg body weight of the subject, i.e., a single dose of 9 mg/kg. Based on a mouse weight of 25 g, each single-dose component I formulation injected into the mice contained 0.225 mg of conjugate 1 (based on siRNA).
(1-1-2) Preparation of component III using conjugate 1: Another conjugate 1 was diluted with a phosphate buffer solution to obtain a 0.6 mg/mL solution (based on the siRNA group), which was designated as component III. Component III was administered at a concentration of 0.6 mg/mL according to a dose of 5 mL/kg body weight of the subject, i.e., a single dose of 3 mg/kg. Based on a mouse weight of 25 g, each single-dose component III formulation injected into the mice contained 0.075 mg of conjugate 1 (based on siRNA).
(1-2) Preparation of immune response regulator in the pharmaceutical composition provided by the present disclosure

Nucleoside monomers were linked one by one in the 3'-5' direction in the order in which nucleotides were arranged according to the sequence shown in SEQ ID NO: 25 by the solid phase phosphoramidite method.
5'-TCGTCGTTTTGTCGTTTTGTCGTT-3' (SEQ ID NO 25)

Each nucleotide was a deoxyribonucleotide. The linking of each nucleoside monomer included a four-step reaction of deprotection, coupling, capping, and sulfurization. Two nucleotides were linked by phosphorothioate. The synthesis conditions are given below:
Nucleoside monomers were provided in an acetonitrile solution with a concentration of 0.1 M. The conditions for the deprotection reaction in each step were the same: the temperature was 25 °C; the reaction time was 70 s; the deprotection reagent was a solution (3% v/v) of dichloroacetic acid in dichloromethane; and the molar ratio of dichloroacetic acid to the 4,4'-dimethoxytrityl protecting group on the solid phase support was 5:1.

The conditions for the coupling reaction in each step were the same, which included: the temperature was 25 °C; the molar ratio of the nucleic acid sequence linked to the solid phase support to the nucleoside monomer was 1:10; the molar ratio of the nucleic acid sequence linked to the solid phase support to the coupling reagent was 1:65; the reaction time was 600 s; the coupling reagent was a 0.5 M solution of 5-(ethylthio)-1H-tetrazole (ETT) in acetonitrile.

The conditions for the capping reaction in each step were the same, which included: the temperature was 25 °C, and the reaction time was 15 s. The capping reagent solution was a mixed solution of CapA and CapB at a molar ratio of 1:1, wherein the CapA and the CapB were capping reagent solutions. The CapA was a mixed solution of 20 vol% N-methylimidazole in pyridine/acetonitrile, and the volume ratio of pyridine to acetonitrile was 3:5. The CapB was a solution of 20 vol% acetic anhydride in acetonitrile. The molar ratio of the capping reagent to the nucleic acid sequence linked to the solid phase support was: acetic anhydride:N-methylimidazole:nucleic acid sequence linked to the solid phase support = 1:1:1.

The conditions for the sulfurization reaction in each step were the same, which included: the temperature was 25 °C; the reaction time was 300 s; the sulfurizing reagent was xanthane hydride. The molar ratio of the sulfurizing reagent to the nucleic acid sequence linked to the solid phase support in the coupling step was 120:1. The reaction was carried out in a mixed solvent of acetonitrile:pyridine = 1:1.

The cleavage and deprotection conditions were as follows: The synthesized nucleotide sequence linked to a support was added to ammonia water with a concentration of 25 wt% in an ammonia water amount of 0.5 mL/µmol. The mixture was reacted at 55 °C for 16 h. The liquid was removed, and the mixture was concentrated to dryness in vacuum.

Purification and desalting: The nucleic acid was purified using a preparative ion chromatography purification column (Source 15Q) through NaCl gradient elution. Specifically, eluent A: 20 mM sodium phosphate (pH 8.1), and the solvent was water/acetonitrile = 9:1 (v/v); eluent B: 1.5 M sodium chloride and 20 mM sodium phosphate (pH 8.1), and the solvent was water/acetonitrile = 9:1 (v/v); elution gradient: eluent A:eluent B = 100:0-50:50 gradient elution. The product eluates were collected, combined, and desalted using a reversed-phase chromatography purification column. The specific conditions included desalting using a Sephadex column (filler: Sephadex-G25), and eluting with deionized water.

Detection: The purity was detected by ion exchange chromatography (IEX-HPLC), and the molecular weight was analyzed by liquid chromatography-mass spectrometry (LC-MS). The measured value was in conformity with the called value, confirming that what was obtained was CpG 7909 compound (component II) shown in SEQ ID NO: 25. Stored at 2-8 °C in the dark, each 50 µg of component II was diluted with a phosphate buffer solution into a 200 µL solution, which was stored separately as 1 part of component II formulation.

### (1-3) Preparation of pharmaceutical composition 1 of the present disclosure

2 parts of component I and 3 parts of component II were combined to prepare pharmaceutical composition 1, wherein each part of component I and each part of component II were stored in different containers. In this case, based on the amount of the siRNA group and the immune response regulator in the siRNA conjugate, the total weight ratio of component I to component II in the prepared pharmaceutical composition 1 was 3:1, and the total dose ratio was 120 mg/kg mouse body weight:1 mg.

### (1-4) Preparation of pharmaceutical composition 2 of the present disclosure

2 parts of component III and 3 parts of component II were combined to prepare pharmaceutical composition 2, wherein each part of component III and each part of component II were stored in different containers. In this case, based on the amount of the siRNA group and the immune response regulator in the siRNA conjugate, the total weight ratio of component III to component II in the prepared pharmaceutical composition 2 was 1:1, and the total dose ratio was 40 mg/kg mouse body weight:1 mg.

### (1-5) Preparation of pharmaceutical composition 3 of the present disclosure

Every 50 µg of alum adjuvant (AH, purchased from Beijing Psaitong Biotechnology Co., Ltd., under the catalog No. A10853) was diluted into a 200 µL solution with a phosphate buffer solution, and stored separately as 1 part of AH formulation.

2 parts of component I, 3 parts of component II, and 3 parts of AH formulation were combined to prepare pharmaceutical composition 4, wherein each part of component I, each part of component II, and each part of AH formulation were stored in different containers. In this case, based on the amount of the siRNA group and the immune response regulator in the siRNA conjugate, the total weight ratio of component I to component II to AH formulation in the prepared pharmaceutical composition 4 was 3:1:1, and the total dose ratio was 180 mg/kg mouse body weight:1 mg:1 mg.

### Experimental Example 1: Anti-hepatitis B virus effect of pharmaceutical composition 1 of the present disclosure in recombinant adeno-associated virus-hepatitis B virus (AAV-HBV) transfected mouse model

The mice used in this experiment were male C57BL/6 mice aged 3-4 weeks with an average body weight of about 25 g, purchased from Shanghai Lingchang Biotechnology Co., Ltd., and transferred from Labcorp experiment 8326405 S529 to enter this experiment. The production license numbers of this batch of animals are SCXK (Shanghai) 2018-0003, and the animal health certificate numbers are 20180003016137 and 20180003016138. The animal use license number of the Labcorp experiment is SYXK (Shanghai) 2021-0001.

The ARCHITECT i2000 (Abbott Laboratories, Lake Bluff, IL, USA) and auxiliary reagents thereof used in this experiment were used for detecting serum hepatitis B surface antigen (HBsAg), hepatitis B e antigen (HBeAg), and hepatitis B surface antibody (HBsAb). Hepatitis B virus DNA (HBV DNA) detection kit was purchased from Sansure Biotech Co., Ltd. (Changsha, Hunan, China), and the PCR instrument (QuantStudio^{™} 3) used was purchased from Applied Biosystems (Foster City, CA, USA).

Conjugate 1 used in this experiment was the sodium salt of conjugate 1 prepared in step (1-1) of Preparation Example 1. During the experiment, the sodium salt of conjugate 1 was diluted with a phosphate buffer solution to the desired concentration and then administered by subcutaneous injection. Immune response regulator 1 used in this experiment was the CpG 7909 sodium salt compound prepared in step (1-2) of Preparation Example 1. During the experiment, the CpG 7909 sodium salt was diluted with a phosphate buffer solution to the desired concentration and then administered by intraperitoneal injection.

The pharmaceutical composition used in this experiment was pharmaceutical composition 1 prepared in Preparation Example 1.

The specific procedures of the experiment are as follows:

### Establishment of AAV-HBV mouse model

[1] To simulate the HBV infection environment, 24 of the above C57BL/6 male mice were acclimated for 14 days, and then each mouse was injected with 200 µL (1 × 10¹¹ vg) of AAV-HBV (purchased from BrainVTA (Wuhan) Co., Ltd., model: rAAV8-1.3HBV (ayw)) via the tail vein. The day of injection was recorded as day 1 of the modeling period. The mice were weighed on day 1, day 22 and day 29 of the modeling period, and serum samples of the mice were collected on day 22 and day 29 of the modeling period. The serum volume collected from each mouse was 15 µL. The collected serum was used for quantitative detection of the hepatitis B virus indexes HBsAg, HBeAg and HBV DNA contents. Based on the measurement values on day 29, mice with qualified blood virology index levels after infection were selected for subsequent treatment experiments. The qualified blood virology index level is a HBsAg level of 4.28 log₁₀ IU/mL-4.90 log₁₀ IU/mL, an HBeAg level of 3.73 log₁₀ S/CO-3.88 log₁₀ S/CO and an HBV DNA level of 6.98 log₁₀ IU/mL-8.64 log₁₀ IU/mL in mouse serum, which are slightly higher than the levels typically observed in human patients with HBV infection to be suitable for assessing long-term efficacy.

The selected 24 mice were randomly divided into 4 groups with 6 mice in each group. During the period from day 1 to the end of the experiment, the health status of the animals was observed twice a day, and the animals were weighed once or twice a week.

The specific administration regimen for each group was as follows:
Group 1: blank control group. On day 1 and day 15, the mice were injected subcutaneously with 0.9% PBS buffer solution at a dose of 5 mL/kg mouse body weight.
Group 2: conjugate control group. On day 1 and day 15, the mice were injected subcutaneously with a solution of conjugate 1 at a concentration of 1.8 mg/mL at a dose of 5 mL/kg mouse body weight. The dose was 9 mpk (mg/kg) per mouse for a single administration.
Group 3: immune response regulator control group in which on days 29, 43, and 57, 50 µg of an immune response regulator solution was injected intraperitoneally at a dose of 200 µL per mouse.
Group 4: pharmaceutical composition 1 group. On day 1 and day 15, the mice were injected subcutaneously with a solution of component I at a concentration of 1.8 mg/mL at a dose of 5 mL/kg mouse body weight. The dose was 9 mpk (mg/kg) per mouse for a single administration. Moreover, on days 29, 43, and 57, the mice were injected intraperitoneally with a solution of component II at 50 µg at a dose of 200 µL per mouse.

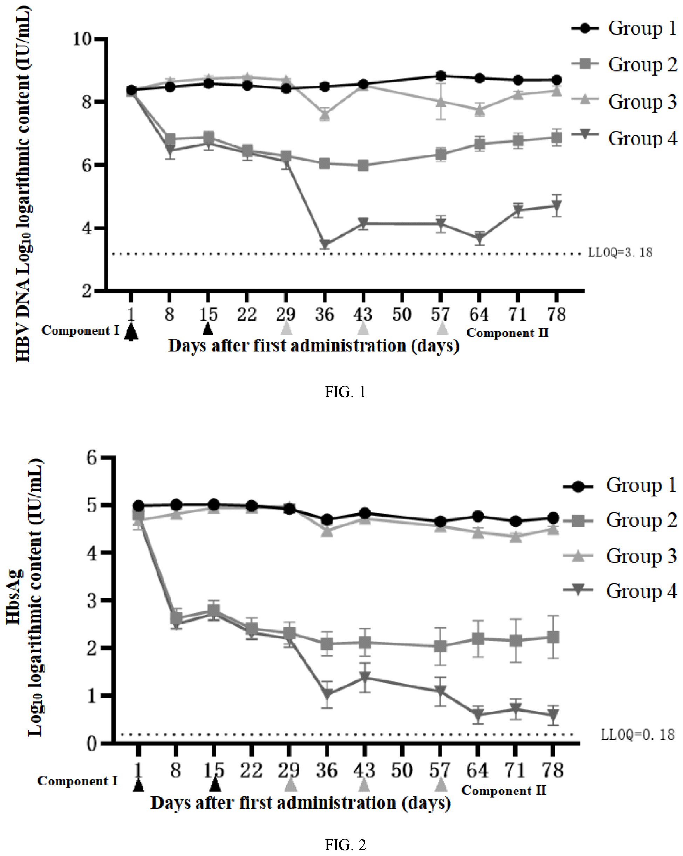

[2] Blood sample collection and quantitative detection of HBV virus-related indexes in the administration period:
Whole blood samples were collected from each mouse on day 1 before administration and on days 8, 15, 22, 29, 36, 43, 57, 64, 71, and 78 to prepare serum for HBV DNA, HBsAg, HBeAg, and HBsAb assays. The experimental results are shown in FIGs. 1-3.

FIG. 1 is a line graph showing the levels of HBV DNA in the serum of mice in groups 1-4 during the administration period. The blank control group (group 1) showed almost no decrease in HBV DNA level. The immune response regulator control group (group 3) showed only a very small decrease in HBV DNA level, with a maximum decrease value of no more than 1 log₁₀ (IU/mL). In another aspect, the conjugate control group (group 2) achieved a maximum decrease of 2.57 log₁₀ IU/mL on day 43 after the administration, i.e., the maximum HBV DNA inhibition rate reached 99.73%.

The pharmaceutical composition 1 group (group 4) of the present disclosure showed a significantly high HBV DNA inhibition effect compared to the control groups described above. The HBV DNA level rapidly further decreased after the administration of component II and was maintained at a low level for up to 78 days after the first administration. The pharmaceutical composition 1 group (group 4) achieved the greatest decrease on day 36, with a decrease of 5.09 log₁₀ IU/mL in HBV DNA, i.e., the maximum HBV DNA inhibition rate reached 99.9992%. Moreover, compared with the control group 2, the pharmaceutical composition of the present disclosure surprisingly further greatly reduced the HBV DNA level by a maximum of 3.01 log₁₀ IU/mL, that is, compared with the use of the RNAi agent alone, the conjugate group further greatly reduced the HBV DNA level by 99.9% after the HBV DNA level had been greatly reduced, and the reduction in the HBV DNA level was far beyond the sum of the inhibition effects of the siRNA conjugate or the immune response regulator alone. Further results showed that in 6 experimental animals in group 4, the HBV DNA level of 1 experimental animal was reduced to the limit of detection (10^{3.18} IU/mL) or less on day 78.

FIG. 2 is a line graph showing changes in the level of HbsAg in the serum of mice in groups 1-4 over time during the administration period, wherein the blank control group (group 1) showed almost no decrease in the level of HbsAg. The immune response regulator control group (group 3) showed only a very small decrease in HBV DNA level, with a maximum decrease value of no more than 0.3 log₁₀ (IU/mL). In another aspect, compared with the blank control group, the conjugate control group (group 2) achieved a maximum decrease of 2.71 log₁₀ IU/mL on day 43 after the administration, i.e., the maximum HbsAg inhibition rate reached 99.80%.

Compared with the control group results described above, similar to the HBV DNA results, the pharmaceutical composition 1 of the present disclosure also surprisingly showed a significantly high HbsAg inhibition effect. The HbsAg level was rapidly reduced after the first administration of component II and was maintained at a relatively low level throughout the 78-day experimental period. The pharmaceutical composition 1 group showed the greatest reduction on day 64, with a 4.17 log₁₀ IU/mL reduction in HbsAg, i.e., the maximum HbsAg inhibition rate reached 99.9930%. Moreover, on day 78 at the end of the experiment, 4.14 log₁₀ IU/mL HbsAg level was still reduced, i.e., the inhibition rate was still 99.9927%. Further, compared with the control group group 2, the pharmaceutical composition 1 of the present disclosure could surprisingly further greatly reduce the HbsAg level by a maximum of 1.65 log ₁₀ IU/mL, that is, compared with the use of the RNAi agent alone, the HbsAg level was further reduced by 97.76% in the conjugate group after it had been greatly reduced, and the reduction was far beyond the sum of the inhibition effects of the siRNA conjugate or the immune response regulator alone. Still further, in 6 experimental animals in group 4, the HbsAg levels in 2 experimental animals were reduced to the limit of detection (10^{0.18} IU/mL) or less after day 57 until the end of the experiment, and the HbsAg level in another experimental animal was reduced to the limit of detection on day 78.

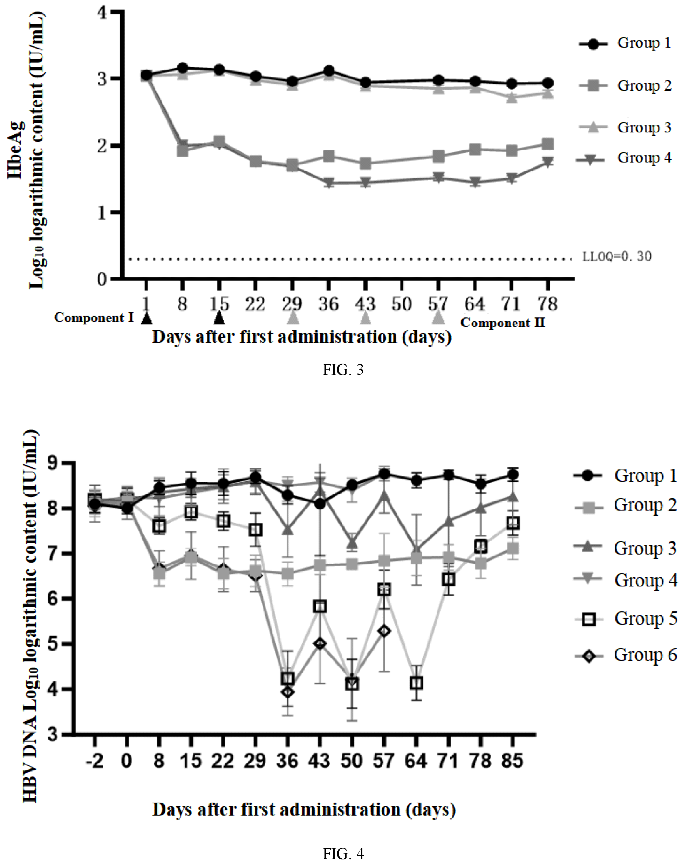

FIG. 3 is a line graph showing changes in the HBeAg level in the serum of mice in groups 1-4 over time during the administration period. The blank control group (group 1) showed almost no decrease in HBeAg level. The immune response regulator control group (group 3) showed only a very small decrease in HBeAg level, with a maximum decrease value of no more than 0.1 log₁₀ (IU/mL). In another aspect, compared with the blank control group, the conjugate control group (group 2) achieved a maximum decrease of about 1 log₁₀ IU/mL on day 43 after the administration, i.e., the maximum HbeAg inhibition rate reached 90%.

The pharmaceutical composition 1 group (group 4) of the present disclosure showed a higher HbeAg inhibition effect compared to the control groups described above, and the HbeAg level showed a further decrease of about 0.3 log₁₀ IU/mL after the first administration of component II. It can be seen that the pharmaceutical composition of the present disclosure could further reduce the HBeAg level compared to the RNAi agent used alone, and the reduction was greater than the sum of the effects of the RNAi agent or the immune response regulator used alone.

In addition, the detection results showed that 3 mice in the pharmaceutical composition 1 group showed a significant increase in the content of HbsAb in serum after the administration, with the highest increases (mIU/mL) of HbsAb separately being 7.76 × 10¹, 2.98 × 10² and 5.72 × 10² (mIU/mL).

Experimental Example 2: Anti-hepatitis B virus effect of pharmaceutical compositions 2-3 of the present disclosure in recombinant adeno-associated virus-hepatitis B virus (AAV-HBV) transfected mouse model

The mice used in this experiment were male C57BL/6 mice aged 3-4 weeks with an average body weight of about 25 g, purchased from Shanghai Lingchang Biotechnology Co., Ltd. The ARCHITECT i2000 (Abbott Laboratories, Lake Bluff, IL, USA) and auxiliary reagents thereof used in this experiment were used for detecting serum hepatitis B surface antigen (HBsAg), hepatitis B e antigen (HBeAg), and hepatitis B surface antibody (HBsAb). Hepatitis B virus DNA (HBV DNA) detection kit was purchased from Sansure Biotech Co., Ltd. (Changsha, Hunan, China), and the PCR instrument (QuantStudio^{™} 3) used was purchased from Applied Biosystems (Foster City, CA, USA).

Conjugate 1 used in this experiment was the sodium salt of conjugate 1 prepared in step (1-1) of Preparation Example 1. During the experiment, the sodium salt of conjugate 1 was diluted with a phosphate buffer solution to the desired concentration and then administered by subcutaneous injection. Immune response regulator 1 used in this experiment was the CpG 7909 sodium salt compound prepared in step (1-2) of Preparation Example 1. During the experiment, the CpG 7909 sodium salt was diluted with a phosphate buffer solution to the desired concentration and then administered by intraperitoneal injection. During the experiment, the AH formulation used in this experiment was diluted with a phosphate buffer solution to the desired concentration and then administered by intraperitoneal injection.

The pharmaceutical compositions 2-3 used in this experiment were pharmaceutical compositions 2-3 prepared in Preparation Example 1.

The specific procedures of the experiment are as follows:

### Establishment of AAV-HBV mouse model

[1] The 42 C57BL/6 male mice described above were modeled according to the procedures in Experimental Example 1. The 42 mouse models were randomly divided into 7 groups with 6 mice in each group. During the period from day 1 to the end of the experiment, the health status of the animals was observed twice a day, and the animals were weighed once or twice a week.

The specific administration regimen for each group was as follows:
Group 1: blank control group. On day 1 and day 15, the mice were injected subcutaneously with 0.9% PBS buffer solution at a dose of 5 mL/kg mouse body weight.
Group 2: conjugate control group. On day 1 and day 15, the mice were injected subcutaneously with a solution of conjugate 1 at a concentration of 1.8 mg/mL at a dose of 5 mL/kg mouse body weight. The dose was 9 mpk (mg/kg) per mouse for a single administration.
Group 3: CpG 7909 control group. On days 29, 43, and 57, 50 µg of a CpG 7909 sodium salt solution was injected intraperitoneally at a dose of 200 µL per mouse.
Group 4: AH formulation control group. On days 29, 43, and 57, 50 µg of an AH formulation solution was injected intraperitoneally at a dose of 200 µL per mouse.
Group 5: pharmaceutical composition 2 group. On day 1 and day 15, the mice were injected subcutaneously with a solution of component III at a concentration of 0.6 mg/mL at a dose of 5 mL/kg mouse body weight. The dose was 3 mpk per mouse for a single administration. Moreover, on days 29, 43, and 57, the mice were injected intraperitoneally with a solution of component II at 50 µg at a dose of 200 µL per mouse.
Group 6: pharmaceutical composition 3 group. On day 1 and day 15, the mice were injected subcutaneously with a solution of component I at a concentration of 1.8 mg/mL at a dose of 5 mL/kg mouse body weight. The dose was 9 mpk per mouse for a single administration. Moreover, on days 29, 43, and 57, the mice were injected intraperitoneally with a solution of component II at 50 µg and a solution of AH formulation at 50 µg at a dose of 200 µL per mouse.

[2] Blood sample collection and quantitative detection of HBV virus-related indexes in the administration period:
For each mouse in groups 1, 2, 3, and 5, whole blood samples were collected 1 day and 3 days before administration and on days 8, 15, 22, 29, 36, 43, 57, 64, 71, 78, and 85 to prepare serum for HBV DNA, HBsAg, HBeAg, and HBsAb assays. For each mouse in groups 4 and 6, whole blood samples were collected 1 day and 3 days before administration and on days 8, 15, 22, 29, 36, 43, and 57 to prepare serum for HBV DNA, HBsAg, HBeAg, and HBsAb assays. The experimental results are shown in FIGs. 4-6.

FIG. 4 is a line graph showing the levels of HBV DNA in the serum of mice in groups 1-6 during the administration period. The blank control group (group 1) and the AH formulation control group (group 4) showed almost no decrease in HBV DNA level. The CpG 7909 control group (group 3) showed only a very small decrease in HBV DNA level, with a maximum decrease value of 1.53 log₁₀ (IU/mL). In another aspect, the conjugate control group (group 2) achieved a maximum decrease of 2 log₁₀ IU/mL on day 22 after the administration, i.e., the maximum HBV DNA inhibition rate reached 99%.

The pharmaceutical composition 2 group (group 5) of the present disclosure showed a significantly high HBV DNA inhibition effect compared to the control groups described above. On day 1 and day 15, component III was administered to the mice once at a dose of 3 mpk. On day 29, after the first administration of component II, the HBV DNA level rapidly decreased and was maintained at a relatively low level for up to 85 days after the first administration. The pharmaceutical composition 2 group (group 5) showed the greatest decrease on day 64, with a decrease of 4.48 log₁₀ IU/mL in HBV DNA, i.e., the maximum HBV DNA inhibition rate reached 99.9967%. Moreover, compared to the conjugate control group with a single dose of the RNAi agent of 9 mpk, pharmaceutical composition 2 of the present disclosure further greatly reduced the HBV DNA level by a maximum of 2.76 log₁₀ IU/mL when the RNAi agent was administered at a single dose of 3 mpk, that is, compared to the RNAi agent used alone, the HBV DNA level was further reduced by 99.83% in the conjugate group after the HBV DNA level had been greatly reduced, and compared to the conjugate control group (group 2), the reduction in the HBV DNA level was far beyond the sum of the inhibition effects of the siRNA conjugate or the immune response regulator alone.

For the pharmaceutical composition 3 group (group 6), component I was administered to the mice once at a dose of 9 mpk on day 1 and day 15. On day 29, after the first administration of the AH formulation and component II, the HBV DNA level rapidly decreased and reached a maximum decrease on day 36, with an HBV DNA decrease of 4.36 log₁₀ IU/mL, i.e., the maximum HBV DNA inhibition rate reached 99.9956%. Compared with the conjugate control group, pharmaceutical composition 3 of the present disclosure further greatly reduced the HBV DNA level, with a maximum reduction of 2.61 log₁₀ IU/mL, that is, the HBV DNA level was further reduced by 99.75% after the HBV DNA level of the conjugate group had been greatly reduced compared to the RNAi agent alone.

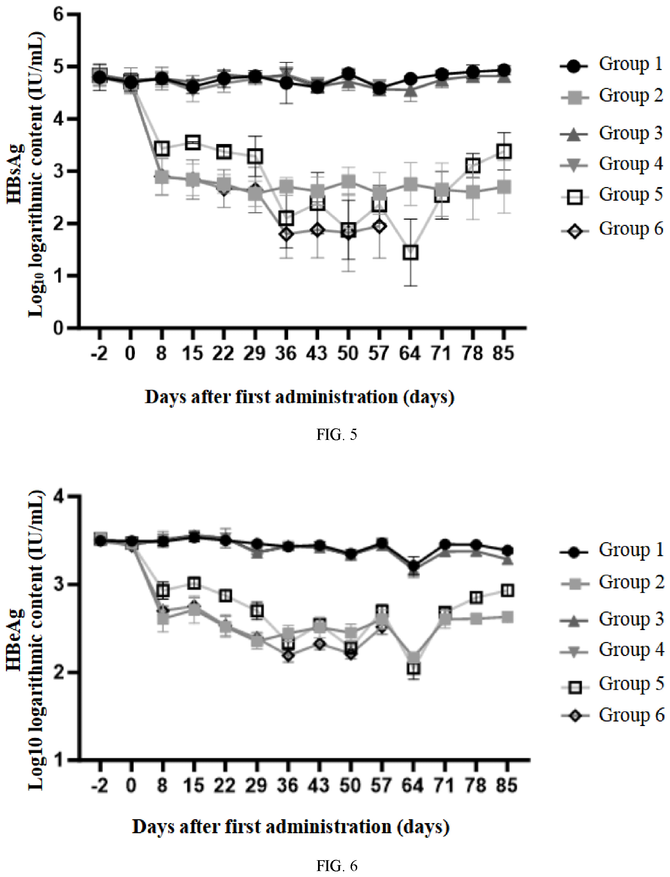

FIG. 5 is a line graph showing changes in the level of HbsAg in the serum of mice in groups 1-6 over time during the administration period, wherein the blank control group (group 1) and the AH formulation control group (group 4) showed almost no decrease in the level of HbsAg. The CpG 7909 control group (group 3) showed only a very small decrease in HBV DNA level, with a maximum decrease value of no more than 0.3 log₁₀ (IU/mL). In another aspect, compared with the blank control group, the conjugate control group (group 2) achieved a maximum decrease of 2.24 log₁₀ IU/mL on day 29 after the administration, i.e., the maximum HbsAg inhibition rate reached 99.4246%.

Compared with the control group results described above, similar to the HBV DNA results, the pharmaceutical composition 2 group (group 5) of the present disclosure also surprisingly showed a significantly high HbsAg inhibition effect. Component III was administered to mice once at a dose of 3 mpk on day 1 and day 15. On day 29, the HbsAg level rapidly decreased after the first administration of component II, and was maintained at a relatively low level for the experimental period of up to 85 days. The pharmaceutical composition 2 group (group 5) achieved the greatest decrease on day 64, with a 3.32 log₁₀ IU/mL decrease in HbsAg, i.e., the maximum HbsAg inhibition rate reached 99.9521%. Further, compared with the conjugate control group group 2, the pharmaceutical composition 2 of the present disclosure could surprisingly further greatly reduce the HbsAg level by a maximum of 1.31 log ₁₀ IU/mL, that is, compared with the use of the RNAi agent alone, the HbsAg level was further reduced by 95.1022% in the conjugate group after it had been greatly reduced. Moreover, the dose of the siRNA conjugate used was significantly reduced, thereby further reducing the drug costs and any safety risks associated with the drug dose.

For the pharmaceutical composition 3 group (group 6), component I was administered to the mice once at a dose of 9 mpk on day 1 and day 15. On day 29, after the first administration of the AH formulation and component II, the HBV DNA level rapidly decreased and reached a maximum decrease on day 36, with an HBV DNA decrease of 2.89 log₁₀ IU/mL, i.e., the maximum HBV DNA inhibition rate reached 99.87%. Compared with the conjugate control group, pharmaceutical composition 3 of the present disclosure further greatly reduced the HBV DNA level, with a maximum reduction of 0.91 log₁₀ IU/mL, that is, the HBV DNA level was further reduced by 87.69% after the HBV DNA level of the conjugate group had been greatly reduced compared to the RNAi agent alone.

FIG. 6 is a line graph showing changes in the HBeAg level in the serum of mice in groups 1-6 over time during the administration period. The blank control group (group 1), the CpG 7909 control group (group 3), and the AH formulation control group (group 4) showed almost no decrease in HBeAg level. Compared with the blank control group, the conjugate control group (group 2) achieved a maximum decrease of about 1.04 log₁₀ IU/mL on day 64 after the administration, i.e., the maximum HbeAg inhibition rate reached 90.88%.

Compared with the control groups described above, the pharmaceutical composition 2 group (group 5) of the present disclosure showed a similar HbeAg inhibition effect to the conjugate control group (group 2). The pharmaceutical composition 3 group (group 6) showed a better HbeAg inhibition effect than that of the conjugate control group. The HbeAg level was further reduced by about 0.325 log₁₀ IU/mL compared to the conjugate control group.

In conclusion, the pharmaceutical composition provided by the present disclosure can induce the production of HBsAb in mice, indicating that the pharmaceutical composition can not only effectively inhibit HBV antigens and DNA, but also stimulate the immune response in mice, showing excellent prospects for achieving a functional cure of hepatitis B.

Some embodiments of the present disclosure have been described in detail above, but the present disclosure is not limited to the specific details of the embodiments. Within the scope of the technical concept of the present disclosure, various simple modifications may be made to the technical solutions of the present disclosure. These simple modifications all belong to the protection scope of the present disclosure.

It should be noted that the various specific technical features described in some embodiments above can be combined in any suitable manner where the features do not contradict each other. In order to avoid unnecessary repetition, such combinations will not be illustrated separately.

In addition, various embodiments of the present disclosure may be combined arbitrarily, as long as they do not violate the idea of the present disclosure, and they should also be regarded as the content disclosed in the present disclosure.

## Claims

1. A pharmaceutical composition, comprising a pharmaceutically active component, wherein the pharmaceutically active component consists of an RNAi agent and an immune response regulator, and the RNAi agent and the immune response regulator exist independently; the RNAi agent refers to one or more of an siRNA composition, an siRNA conjugate, and a pharmaceutically acceptable salt thereof; the siRNA composition comprises an siRNA and a pharmaceutically acceptable carrier; the siRNA conjugate comprises an siRNA group and a conjugating group conjugatively linked to the siRNA group; the siRNA group refers to a group formed by removing one or more atoms or groups from the siRNA, and the siRNA is an siRNA capable of inhibiting HBV mRNA; based on siRNA, the weight ratio of the RNAi agent to the immune response regulator is (0.5-5000):1.

2. The pharmaceutical composition according to claim 1, wherein the weight ratio of the RNAi agent to the immune response regulator is (0.5-2000):1, (1-400):1, (2-150):1, or (2.4-50):1.

3. The pharmaceutical composition according to claim 1, wherein based on siRNA, the dose ratio of the RNAi agent to the immune response regulator is (0.02-540) mg/kg body weight of a subject:1 mg, (0.03-400) mg/kg body weight of a subject:1 mg, (0.06-380) mg/kg body weight of a subject:1 mg, (0.06-180) mg/kg body weight of a subject:1 mg, (0.1-80) mg/kg body weight of a subject:1 mg, or (0.2-30) mg/kg body weight of a subject:1 mg.

4. The pharmaceutical composition according to claim 1, wherein the RNAi agent refers to an siRNA conjugate or a pharmaceutically acceptable salt thereof, the conjugating group comprises a pharmaceutically acceptable targeting group and a linker, and the siRNA group, the linker and the targeting group are linked sequentially.

5. The pharmaceutical composition according to claim 4, wherein the RNAi agent refers to an siRNA conjugate having a structure represented by formula (308) or a pharmaceutically acceptable salt thereof: 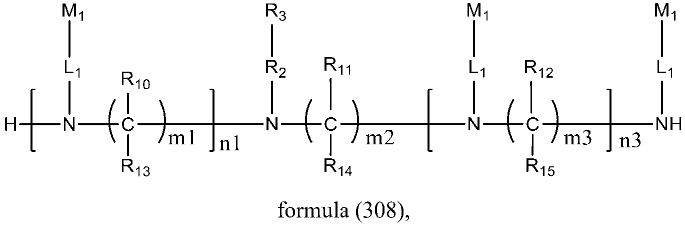 wherein,
n1 is an integer selected from 1-3, and n3 is an integer selected from 0-4;
each m1, m2, or m3 is independently an integer selected from 2-10;
R₁₀, R₁₁, R₁₂, R₁₃, R₁₄ or R₁₅ are each independently H, or are selected from the group consisting of the following groups: C₁-C₁₀ alkyl, C₁-C₁₀ haloalkyl and C₁-C₁₀ alkoxy;
R₃ has a structure represented by formula (A59): 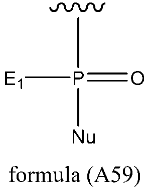
wherein E₁ is OH, SH or BH₂, and Nu represents the siRNA group;
R₂ is a linear alkylene group with a length of 1-20 carbon atoms, wherein one or more carbon atoms are optionally replaced by any one or more selected from the group consisting of the following groups: C(O), NH, O, S, CH=N, S(O)₂, C₂-C₁₀ alkenylene, C₂-C₁₀ alkynylene, C₅-C₁₀ cyclohydrocabylene, C₆-C₁₀ arylene, C₃-C₁₈ heterocyclylene and C₅-C₁₀ heteroarylene; and wherein R₂ may optionally have any one or more substituents from the group consisting of the following groups: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halo substituents, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(0)O(C₁-C₁₀ alkyl), -CON(C₁-C₁₀ alkyl)(C₁-C₁₀ alkyl), - CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
each L₁ is independently a linear alkylene group with a length of 1-70 carbon atoms, wherein one or more carbon atoms are optionally replaced by any one or more selected from the group consisting of the following groups: C(O), NH, O, S, CH=N, S(O)₂, C₂-C₁₀ alkenylene, C₂-C₁₀ alkynylene, C₆-C₁₀ arylene, C₃-C₁₈ heterocyclylene and C₅-C₁₀ heteroarylene; and wherein L₁ may optionally have any one or more substituents from the group consisting of the following groups: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halo substituents, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(0)O(C₁-C₁₀ alkyl), -CON(C₁-C₁₀ alkyl)(C₁-C₁₀ alkyl), - CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
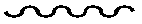 indicates the site where the group is covalently linked;
each M₁ is independently selected from one of ligands that have an affinity for the asialoglycoprotein receptor on the surface of mammalian hepatocytes.

6. The pharmaceutical composition according to claim 5, wherein the RNAi agent refers to a conjugate having a structure represented by formula (403) or a pharmaceutically acceptable salt thereof: 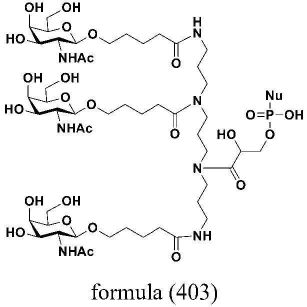 wherein Nu represents the siRNA group.

7. The pharmaceutical composition according to claim 4, wherein the RNAi agent refers to a conjugate having a structure represented by formula (305) or a pharmaceutically acceptable salt thereof: 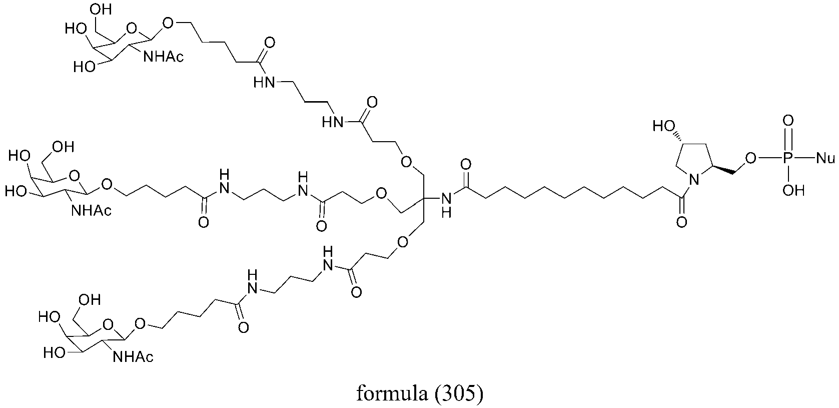 wherein Nu represents the siRNA group.

8. The pharmaceutical composition according to claim 1, wherein the siRNA composition comprises an siRNA, a critical lipid, a helper lipid, and a pegylated lipid, wherein the critical lipid has a structure represented by formula (214) or formula (215): 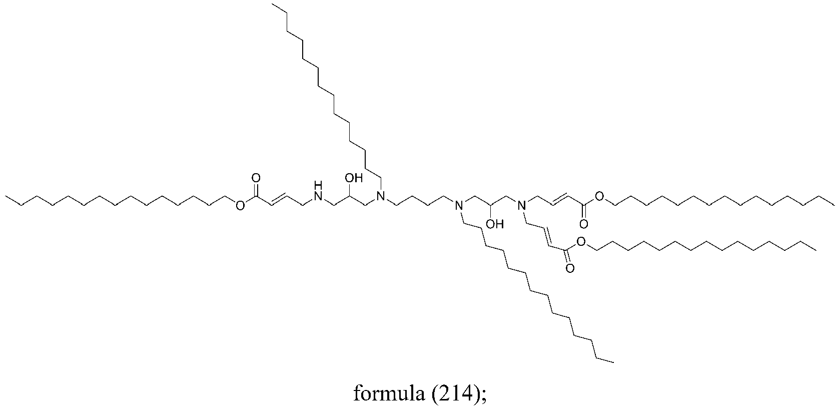 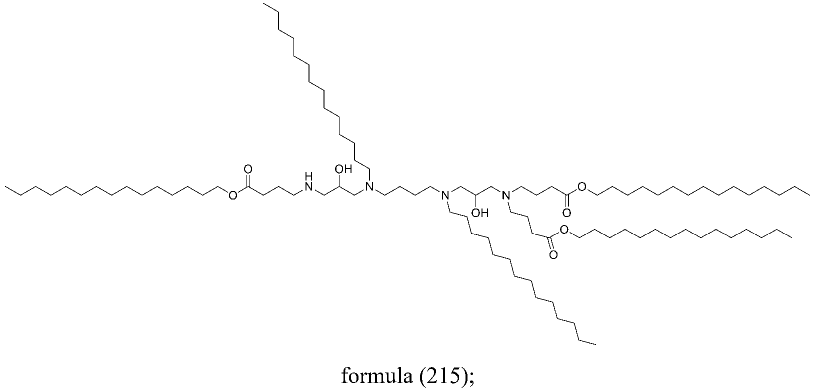
the helper lipid is a cholesterol, a cholesterol analog and/or a cholesterol derivative;
the pegylated lipid is 1,2-dipalmitoyl-sn-glycero-3-phosphatidylethanolamine-N-[methoxy(polyethylene glycol)]-2000;
the molar ratio among the organic amine, the helper lipid and the pegylated lipid is (19.7-80) : (19.7-80) : (0.3-50); or the molar ratio among the organic amine, the helper lipid and the pegylated lipid is (50-70) : (20-40) : (3-20).

9. The pharmaceutical composition according to claim 1, wherein the siRNA comprises a sense strand and an antisense strand, the sense strand comprises a nucleotide sequence I, and the antisense strand comprises a nucleotide sequence II, wherein the nucleotide sequence I and the nucleotide sequence II each consist of 19 nucleotides, each of the nucleotides in the nucleotide sequence I and the nucleotide sequence II is a modified or unmodified nucleotide, the nucleotide sequence I and the nucleotide sequence II are at least partially reverse complementary to form a double-stranded region, the nucleotide sequence II is at least partially reverse complementary to a first nucleotide sequence segment, and the first nucleotide sequence segment is a nucleotide sequence of 19 nucleotides in length in HBV mRNA.

10. The pharmaceutical composition according to claim 9, wherein the sense strand and the antisense strand are identical or different in length; the sense strand is 19-23 nucleotides in length, and the antisense strand is 20-26 nucleotides in length.

11. The pharmaceutical composition according to claim 9, wherein the nucleotide sequence I has the same length and no more than three nucleotide differences from the nucleotide sequence shown in SEQ ID NO: 1; and the nucleotide sequence II has the same length and no more than three nucleotide differences from the nucleotide sequence shown in SEQ ID NO: 2:
5'-CCUUGAGGCAUACUUCAAZ₁-3' (SEQ ID NO: 1);
5'-Z₂UUGAAGUAUGCCUCAAGG-3' (SEQ ID NO: 2);
wherein Z₁ is A, Z₂ is U, the nucleotide sequence I comprises a nucleotide Z₃ at a corresponding site to Z₁, the nucleotide sequence II comprises a nucleotide Z₄ at a corresponding site to Z₂, and Z₄ is the first nucleotide at the 5' terminal of the antisense strand;
or the nucleotide sequence I has the same length and no more than three nucleotide differences from the nucleotide sequence shown in SEQ ID NO: 3; and the nucleotide sequence II has the same length and no more than three nucleotide differences from the nucleotide sequence shown in SEQ ID NO: 4:
5'-GUGUGCACUUCGCUUCACZ₅-3' (SEQ ID NO: 3);
5'-Z₆GUGAAGCGAAGUGCACAC-3' (SEQ ID NO: 4);
wherein Z₅ is A, Z₆ is U, the nucleotide sequence I comprises a nucleotide Z₇ at a corresponding site to Z₅, the nucleotide sequence II comprises a nucleotide Z₈ at a corresponding site to Z₆, and Z₈ is the first nucleotide at the 5' terminal of the antisense strand;
or the nucleotide sequence I has the same length and no more than three nucleotide differences from the nucleotide sequence shown in SEQ ID NO: 5; and the nucleotide sequence II has the same length and no more than three nucleotide differences from the nucleotide sequence shown in SEQ ID NO: 6:
5'-GGACUUCUCUCAAUUUUCZ₉-3' (SEQ ID NO: 5);
5'-Z₁₀GAAAAUUGAGAGAAGUCC-3' (SEQ ID NO: 6);
wherein Z₉ is U, Z₁₀ is A, the nucleotide sequence I comprises a nucleotide Z₁₁ at a corresponding site to Z₉, the nucleotide sequence II comprises a nucleotide Z₁₂ at a corresponding site to Z₁₀, and Z₁₂ is the first nucleotide at the 5' terminal of the antisense strand;
or the nucleotide sequence I has the same length and no more than three nucleotide differences from the nucleotide sequence shown in SEQ ID NO: 7; and the nucleotide sequence II has the same length and no more than three nucleotide differences from the nucleotide sequence shown in SEQ ID NO: 8:
5'-CUGUAGGCAUAAAUUGGUZ₁₃-3' (SEQ ID NO: 7);
5'-Z₁₄ACCAAUUUAUGCCUACAG-3' (SEQ ID NO: 8);
wherein Z₁₃ is A, Z₁₄ is U, the nucleotide sequence I comprises a nucleotide Z₁₅ at a corresponding site to Z₁₃, the nucleotide sequence II comprises a nucleotide Z₁₆ at a corresponding site to Z₁₄, and Z₁₆ is the first nucleotide at the 5' terminal of the antisense strand;
or the nucleotide sequence I has the same length and no more than three nucleotide differences from the nucleotide sequence shown in SEQ ID NO: 9; and the nucleotide sequence II has the same length and no more than three nucleotide differences from the nucleotide sequence shown in SEQ ID NO: 10:
5'-GUGCACUUCGCUUCACZ₁₇-3' (SEQ ID NO: 9);
5'-Z₁₈ACCAAUUUAUGCCUACAG-3' (SEQ ID NO: 10);
wherein Z₁₇ is A, Z₁₈ is U, the nucleotide sequence I comprises a nucleotide Z₁₉ at a corresponding site to Z₁₇, the nucleotide sequence II comprises a nucleotide Z₂₀ at a corresponding site to Z₁₈, and Z₂₀ is the first nucleotide at the 5' terminal of the antisense strand.

12. The pharmaceutical composition according to claim 1, wherein the immune response regulator is selected from one or more of an adjuvant and an immunostimulant, wherein the adjuvant is selected from one or more of a reagent capable of promoting an immune response, and the immunostimulant is selected from one or more of an independently administrable reagent capable of stimulating an immune response;
or the adjuvant is selected from a pathogen component, a particulate adjuvant, and a combined adjuvant;
or the pathogen component is selected from monophosphoryl lipid A (MPL), poly(I:C), poly ICLC adjuvant, CpG DNA, c-di-AMP, c-di-GMP, and c-di-CMP; short and blunt-ended 5'-triphosphate dsRNA (3pRNA) RIG-1 ligand, and emulsion;
or the particulate adjuvant is selected from alum, virosomes, and cytokines.

13. The pharmaceutical composition according to claim 12, wherein the immune response regulator is selected from one or more of TLR agonists; or the immune response regulator is selected from one or more of TLR9 agonists.

14. The pharmaceutical composition according to claim 13, wherein the immune response regulator is selected from one or more of alum adjuvant, CpG DNA, and a pharmaceutically acceptable salt thereof; or the CpG DNA comprises the nucleotide sequence shown in SEQ ID NO 25 or SEQ ID NO 26:
5'-TCGTCGTTTTGTCGTTTTGTCGTT-3' (SEQ ID NO 25)
5'-TGACTGTGAACGTTCGAGATGA-3' (SEQ ID NO 26);
wherein each nucleotide in the CpG DNA is a deoxynucleotide, and the nucleotides in the CpG DNA are linked by a phosphorothioate ester bond.

15. The pharmaceutical composition according to claim 1, wherein the RNAi agent is a conjugate having a structure represented by formula (403), a sodium salt thereof, or a partial sodium salt thereof, wherein Nu represents an siRNA group, the siRNA group has a sense strand shown in SEQ ID NO: 11 and an antisense strand shown in SEQ ID NO: 12, and the siRNA group is formed by removing one hydrogen atom from the 3' hydroxy of the 3' terminal nucleotide of the sense strand;
5'-CmsCmsUmUmGmAmGfGfCfAmUmAmCmUmUmCmAmAmAm-3' (SEQ ID NO: 11);
5'-VP-UmsUfsUmGmAmAfGmUmAmUmGmCmCmUfCmAfAmGmGmsUmsUm-3' (SEQ ID NO: 12);
the immune response regulator is a CpG DNA shown in SEQ ID NO: 25, a sodium salt thereof or a partial sodium salt thereof, and/or an alum adjuvant.

16. The pharmaceutical composition according to claim 1, wherein the pharmaceutical composition further comprises an auxiliary agent, wherein the auxiliary agent forms a pharmaceutical formulation with the RNAi agent and/or the immune response regulator, and the auxiliary agent is selected from one or more of a solvent, a pharmaceutically acceptable carrier, and a pharmaceutically acceptable excipient;
or, based on siRNA in the RNAi agent, the weight ratio of the RNAi agent to the auxiliary agent is 1:(1-600) or 1:(1-50); the weight ratio of the immune response regulator to the auxiliary agent is 1:(1-5000) or 1:(1-500).

17. The pharmaceutical composition according to any one of claims 1-16, wherein a pharmaceutically acceptable salt of one or more of the siRNA, the siRNA conjugate, and the CpG DNA is a water-soluble salt or partial salt; or the pharmaceutically acceptable salt is an alkali metal salt or a partial alkali metal salt.

18. The use according to claim 1, wherein the RNAi agent is present in a formulation for subcutaneous injection, and the immune response regulator is present in a formulation for intraperitoneal injection or subcutaneous injection.

19. Use of the pharmaceutical composition according to any one of claims 1-18 in preparing a medicament for treating a disease associated with hepatitis B virus infection.

20. The use according to claim 19, wherein the disease associated with hepatitis B virus infection is one or more of inflammation caused by hepatitis B virus infection, hepatic fibrosis, liver proliferative disease, liver failure, and hepatocellular carcinoma; or the inflammation caused by hepatitis B virus infection refers to hepatitis B and/or hepatitis D.

21. A method for treating a disease associated with hepatitis B virus infection, comprising administering to a subject an effective amount of the pharmaceutical composition according to any one of claims 1-17.

22. The method according to claim 21, wherein the method comprises one or more treatment courses, wherein the RNAi agent and the immune response regulator are each independently administered one or more times in one treatment course;
or, in one treatment course, after an effective amount of the RNAi agent is administered to the subject, the immune response regulator is first administered; or, in one treatment course, after 0.01-27 mg/kg of the RNAi agent is administered to the subject, 0.03-3 mg of the immune response regulator is first administered;
or, in one treatment course, the subject is administered 0.1-9 mg/kg body weight of the subject of the RNAi agent each time in 1-5 administrations, followed by 1-5 administrations of 0.05-1 mg of the immune response regulator each time.

23. The method according to claim 22, wherein based on siRNA, the dose of the RNAi agent in a single administration is 0.05 mg-200 mg, and the dose of the immune response regulator in a single administration is 0.05-2 mg;
or, based on siRNA, the dose of the RNAi agent in a single administration is 4 mg-200 mg, and the dose of the immune response regulator in a single administration is 0.1 mg-2 mg; or, based on siRNA, the dose of the RNAi agent in a single administration is 6 mg-180 mg, and the dose of the immune response regulator in a single administration is 0.12 mg-1 mg.

24. The method according to claim 22, wherein based on siRNA, the dose of the RNAi agent in a single administration is 0.05-3.5 mg/kg body weight of the subject, and the dose of the immune response regulator in a single administration is 0.1-2 mg; or, based on siRNA, the dose of the RNAi agent in a single administration is 0.1-3 mg/kg body weight of the subject, and the dose of the immune response regulator in a single administration is 0.12-1 mg;
or, based on siRNA, the dose of the RNAi agent in a single administration is 2.5-12 mg/kg body weight of the subject, and the dose of the immune response regulator in a single administration is 20-200 µg; or, based on siRNA, the dose of the RNAi agent in a single administration is 3-9 mg/kg body weight of the subject, and the dose of the immune response regulator in a single administration is 30-50 µg.

25. The method according to any one of claims 22-24, wherein the administration is performed multiple times, with an interval of 5 days to 60 weeks between each administration of the RNAi agent, and with an interval of 1 day to 60 days between each administration of the immune response regulator; or
the interval between each administration of the RNAi agent is 10 days to 40 weeks, and the interval between each administration of the immune response regulator is 5 days to 45 days; or
the immune response regulator is administered after administration of an effective amount of the RNAi agent, wherein the RNAi agent and the immune response regulator are administered at an interval of 5 days to 2 months, or the RNAi agent and the immune response regulator are administered at an interval of 7 days to 45 days.

26. The method according to claim 23, wherein a single dose of the immune response regulator is administered first after administration of 12 mg-200 mg of the RNAi agent; or a single dose of the immune response regulator is administered first after administration of 0.3 mg-0.5 mg of the RNAi agent.

27. The method according to any one of claims 21-26, wherein the disease associated with hepatitis B virus infection is one or more of inflammation caused by hepatitis B virus infection, hepatic fibrosis, liver proliferative disease, liver failure, and hepatocellular carcinoma; or the inflammation caused by hepatitis B virus infection refers to hepatitis B and/or hepatitis D.

28. A kit, comprising the pharmaceutical composition according to any one of claims 1-17.
